(19) Europäisches Patentamt
European Patent Office
Office européen des brevets

(11) Publication number: 0 007 156
B1

(12) EUROPEAN PATENT SPECIFICATION

(45) Date of publication of patent specification: 21.07.82

(51) Int. Cl.³: C 07 C 145/04, A 01 N 47/08

(21) Application number: 79300732.9

(22) Date of filing: 30.04.79

# (54) Carbamates, compositions containing them, their insecticidal and nematocidal use, and a method for their preparation.

(30) Priority: 02.05.78 US 902197

(43) Date of publication of application: 23.01.80 Bulletin 80/2

(45) Publication of the grant of the patent: 21.07.82 Bulletin 82/29

(84) Designated Contracting States: BE DE FR GB IT LU NL

(56) References cited:
FR - A - 2 333 788

(73) Proprietor: E.I. DU PONT DE NEMOURS AND COMPANY
Legal Department 1007 Market Street
Wilmington Delaware 19898 (US)

(72) Inventor: Hay, James Volney
36 Stature Drive Sherwood Forest
Newark Delaware 19713 (US)

(74) Representative: Hildyard, Edward Martin et al,
Frank B. Dehn & Co. Imperial House 15-19
Kingsway
London WC2B 6UZ (GB)

EP 0 007 156 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

# Carbamates, compositions containing them, their insecticidal and nematocidal use, and a method for their preparation

This invention relates to insecticidal and nematicidal carbamates, and preparation and use thereof.

Insecticidal carbamates are known in the prior art, e.g.

(a) Belgian BE 848,911 discloses compounds of the formula

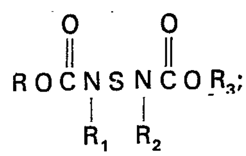

(b) Belgian BE 848,914 discloses compounds of the formula

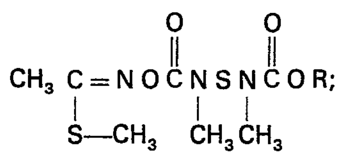

(c) Belgian BE 855,928 discloses compounds of the formula

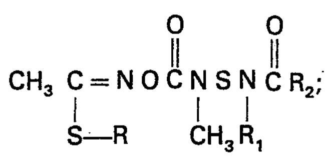

(d) German DT 2,654,246 discloses compounds of the formula

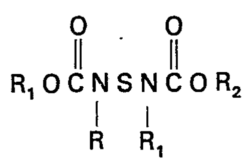

In the above publications the R substituents are widely defined.

## SUMMARY OF THE INVENTION

This invention relates to novel compounds of Formula I, to agriculturally useful compositions of these, and to the method of use of these compounds as insecticides and nematocides.

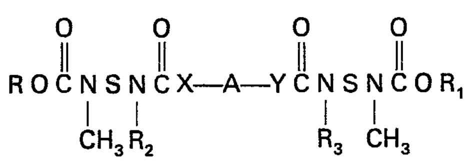 I

where R is 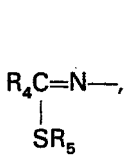, 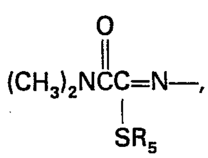, or 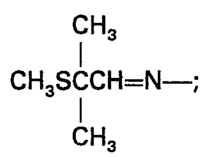; $R_1$ is 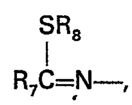, 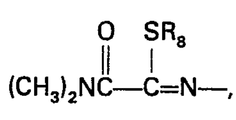, or 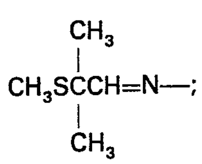;

$R_2$, $R_3$, $R_5$ and $R_8$ are independently $C_1$—$C_3$ alkyl;
$R_4$ and $R_7$ are independently $C_1$—$C_3$ alkyl or $CH_3OCH_2$—;
A is —$(CT_1T_2)_k(CT_3T_4)_l(CT_5T_6)_m$;

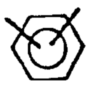 ; 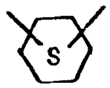 ; 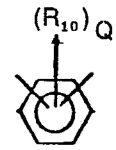 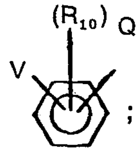 ; 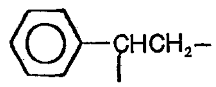 ; 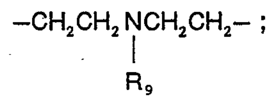 ;

or $-CT_1T_2CT_3T_4 -[- OCT_1T_2CT_3T_4 -]_p OCT_1T_2CT_3T_4-$ ;

X and Y are independently $NR_6$, O or S;
$T_1$ to $T_6$ are independently hydrogen or $C_1$—$C_2$ alkyl;
$R_6$ is H or alkyl $C_1$—$C_3$;

$R_9$ is alkyl $C_1$—$C_3$, 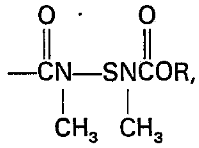 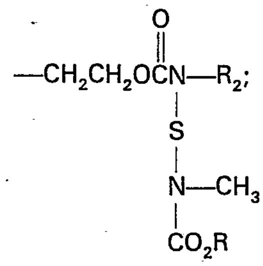

V is O, $S(O)_n$, $CT_7T_8$;
$R_{10}$ is H, alkyl of $C_1$—$C_3$, Cl;
$T_7$ is H, $C_1$—$C_2$ alkyl, carboalkoxy alkyl of 3—8 carbon atoms, $C_2$—$C_4$ carboalkoxy;
$T_8$ is H, alkyl $C_1$—$C_4$;
$T_7$ and $T_8$ may be taken together to form a carbocyclic ring of 5—6 carbons;
Q is 0—2;
k, l, m are independently 0—3;
n, p are independently 0—2,

provided

a) the sum of k, l and m is at least two;
b) the total carbon content of —$(CT_1T_2)_k$; $(CT_3T_4)_l$ $(CT_5T_6)_m$— will not exceed g;
c) when A is 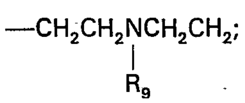 then X and Y are both oxygen and $R=R_1$;
d) when A is $CT_1T_2CT_3T_4 -[OCT_1T_2CT_3Y_4]_p$ —$OCT_1T_2CT_3T_4$, X and Y are both oxygen.

## DETAILED DESCRIPTION OF THE INVENTION

### *Preferred Compounds*

Preferred for their ease of synthesis and/or favorable cost are compounds of Formula I wherein $R=R_1$.

More preferred for their higher activity and/or more favorable cost are compounds of Formula I wherein $R=R_1$; and

R and $R_1$= 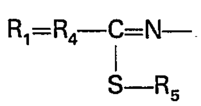 or 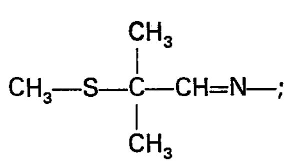 ;

X and Y are both oxygen;

A is $(CT_1T_2)_k$ $(CT_3T_4)_l$ $(CT_5T_6)_m$ or 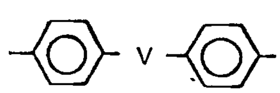 ; and

k, l and m are independently 0—3.

Even more preferred are compounds within the preceding scope wherein

R and 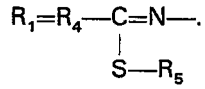

Most preferred for their excellent activity and/or most favorable cost are compounds within the even more preferred scope wherein

$R_2$, $R_3$ and $R_5$ are methyl;

$R_4$ is $C_1$—$C_2$ alkyl;

A is $(CT_1T_2)_k$ $(CT_3T_4)_l$ $(CT_5T_6)_m$ or 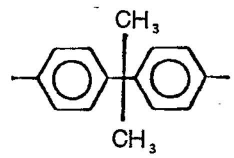 ; and

$T_1$—$T_6$ are hydrogen;

provided the sum of k, l, and m total not more than 5.

*Specific Examples are:*

dimethyl N,N′ - [[1,2 - ethanediylbis[oxycarbonyl(N - methylimino)thio(N - methylimino)-carbonyloxy]]]bis - [ethanimidothioate];

dimethyl N,N′ - [[1,3 - propanediylbis[oxycarbonyl(N - methylimino)thio(N - methylimino)-carbonyloxy]]]bis - [ethanimidothioate]; and

dimethyl N,N′[[2,2 - propanediylbis[4,1 - phenyleneoxycarbonyl(N - methylimino)thio(N-methylimino)carbonyloxy]]]bis - [ethanimidothioate].

*Methods of Preparation*

The compounds of Formula I wherein R = $R_1$ can be prepared, as shown in Equation A, by reacting a bissulfenyl chloride of Formula II with two mole equivalents of a carbamate of Formula III, in the presence of an acid acceptor.

EQUATION A

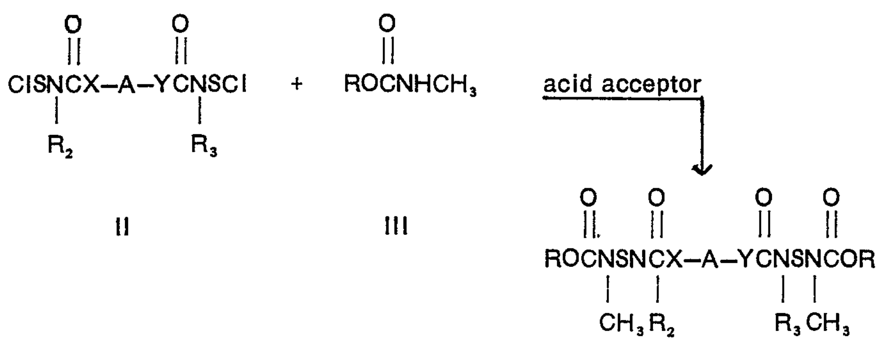


II III I

wherein R, $R_2$, $R_3$, X, Y, and A are as previously defined.

The reaction represented in Equation A can be carried out in an inert organic solvent such as benzene, toluene, the xylenes, ethyl acetate, methylene chloride, chloroform, ethylene dichloride, tetrahydrofuran, dioxane, or dimethylformamide. Mixtures of these solvents may be used. The reaction can be performed at temperatures between −15° and 50°C, preferably between −5°C and 30°C. Pressure is not critical for the reaction procedure since pressures above and below atmospheric are suitable. For convenience, atmospheric pressure is preferred.

The acid acceptor used in Equation A can be a tertiary organic amine, such as, trimethylamine, triethylamine, N,N-dimethylaniline, or pyridine.

The compounds of Formula I obtained via the reaction shown in Equation A may be purified by methods known to those skilled in the art, such as recrystallization, column chromatography, or another suitable procedure.

The compounds of Formula I wherein $R \neq R_1$ can be prepared as shown in Equation B by reacting a bissulfenyl chloride of Formula II with one mole equivalent of a carbamate of Formula III and one mole equivalent of a carbamate of Formula IV, in the presence of an acid acceptor.

EQUATION B

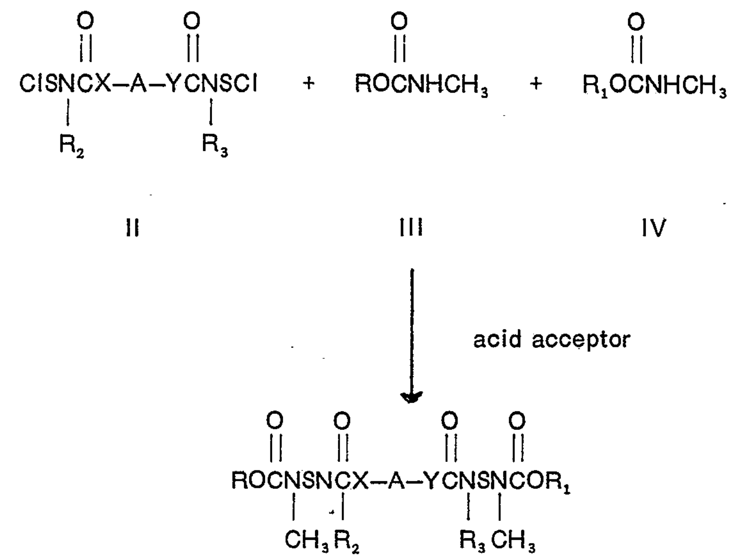


II III IV

I

wherein R, $R_1$, $R_2$, $R_3$, X, Y, and A are as previously defined.

The reaction shown in Equation B can be carried out in an inert organic solvent, such as, benzene, toluene, the xylenes, ethyl acetate, methylene chloride, chloroform, ethylene dichloride, tetrahydrofuran, dioxane or dimethylformamide. Mixtures of these solvents may be used. The reaction can be carried out at temperatures between −15° and 50°C, preferably between −5° and 30°C. Pressure is not critical for the reaction procedure since pressures above and below atmospheric are suitable. For convenience, atmospheric pressure is preferred.

The acid acceptor employed in Equation B can be tertiary organic amines, such as, trimethylamine, triethylamine, N,N-dimethylaniline, or pyridine.

The compounds of Formula I obtained by the reaction shown in Equation B can be purified by suitable procedures, such as, high-pressure liquid chromatography.

The bissulfenyl chlorides of Formula II used as intermediates in Equation A and Equation B can be prepared, as shown in Equation C, by reacting a bisaminocarbonyl compound of Formula V with two mole equivalents of sulfur dichloride in the presence of an acid acceptor.

EQUATION C

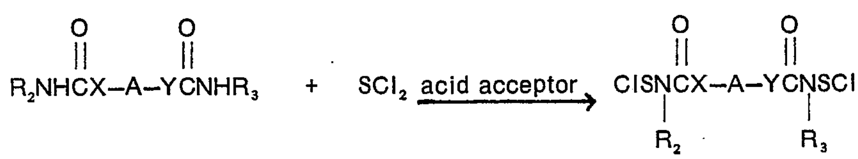


V II

wherein $R_2$, $R_3$, X, Y, and A are as previously defined.

The reaction shown in Equation C can be carried out in an inert organic solvent, such as benzene, toluene, ethyl acetate, methylene chloride, chloroform, or ethylene dichloride. Mixtures of these solvents may be used. The reaction can be carried out at temperatures between −25° and 15°C, preferably between −10° and 5°C. Pressure is not critical for the reaction procedure since pressures above and below atmospheric are suitable. For convenience, atmospheric pressure is preferred.

The acid acceptor used in Equation C can be a tertiary organic amine, such as, trimethylamine, triethylamine, N,N-dimethylaniline, or pyridine.

The carbamates of Formula III and Formula IV employed as reactants in Equation A and Equation

B can be prepared by the methods taught in U.S. 3,576,834, U.S. 3,530,220, and U.S. 3,217,037.

The compounds of Formula I wherein $R = R_1$ and $R_2 = R_3 = CH_3$ can also be prepared, as shown in Equation D, by reacting a compound of Formula VI with two mole equivalents of a carbamyl fluoride of Formula VII, in the presence of an acid acceptor.

EQUATION D

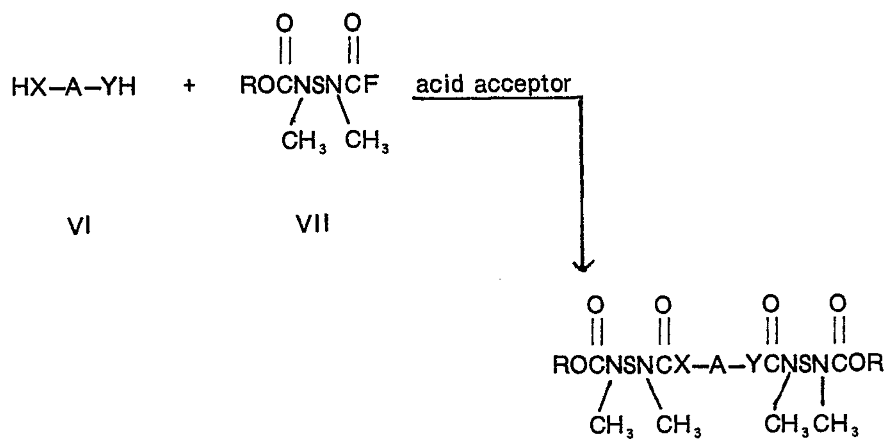


VI VII

I

wherein R, X, Y, and A are as previously defined.

The reaction shown in Equation D can be carried out in an inert organic solvent, such as benzene, toluene, the xylenes, methylene chloride, chloroform, ethylene dichloride, acetone, methyl ethyl ketone, dioxane, tetrahydrofuran, acetonitrile, or dimethylformamide. Mixtures of these solvents may be used. The reaction can be carried out at temperatures between 0° and 150°C, preferably between 20° and 100°C. Pressure is not critical for the reaction procedure since pressures above and below atmospheric are suitable. For convenience, atmospheric pressure is preferred.

The acid acceptor used in Equation D can be a tertiary organic amine, such as, trimethylamine, triethylamine, N,N-dimethylaniline, or pyridine, or inorganic bases, such as, the alkali metal and alkaline earth metal hydroxides, carbonates, bicarbonates, and alkoxides, such as, sodium methoxide or potassium *tert*-butoxide.

The compounds of Formula I obtained by the reaction described in Equation D can be purified by methods known to those skilled in the art, such as, recrystallization, column chromatography or another suitable procedure.

Carbamyl fluorides of Formula VII can be prepared by the method taught in DT 2,654,246 and BE 848,914.

The compounds of Formula I wherein $R = R_1$ and $R_2 = R_3 = CH_3$ can also be prepared, as shown in Equation E, by reacting a bis-carbamyl fluoride of Formula VIII with two mole equivalents of an oxime of Formula IX, in the presence of an acid acceptor.

EQUATION E

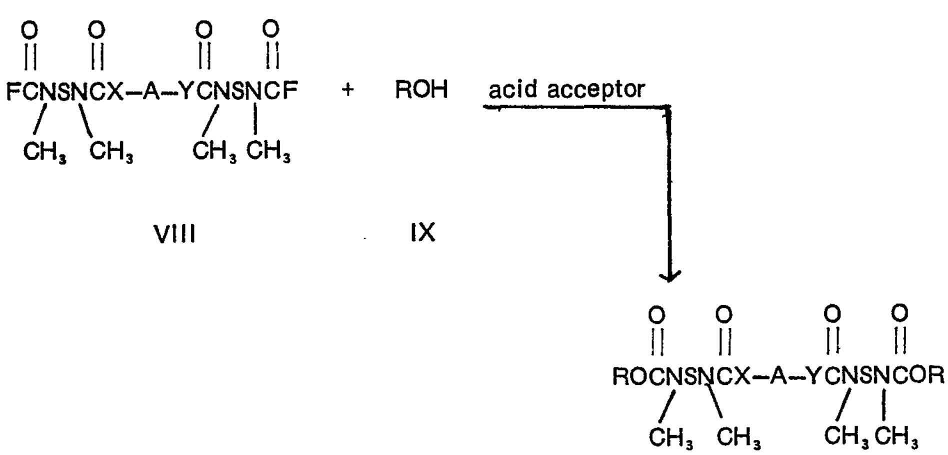


VIII IX

I

wherein R, X, Y, and A are as previously defined.

The reaction shown in Equation E can be carried out in inert organic solvents such as benzene, toluene, the xylenes, methylene chloride, chloroform, ethylene dichloride, acetone, methyl ethyl ketone, dioxane, tetrahydrofuran, acetonitrile, or dimethylformamide. Mixtures of these solvents may be used. The reaction can be carried out at temperatures between 0° and 100°C, preferably between 20° to 50°C. Pressure is not critical for the reaction procedure since pressures above and below atmospheric are suitable. For convenience, atmospheric pressure is preferred.

The acid acceptor employed in Equation E can be tertiary organic amines, such as, trimethylamine, triethylamine, N,N-dimethylaniline, or pyridine, or inorganic bases, such as, the alkali metal and alkaline earth metal hydroxides, carbonates, bicarbonates, and alkoxides, such as, sodium methoxide or potassium *tert*-butoxide.

The compounds of Formula I wherein $R \neq R_1$, $R_2 = R_3 = CH_3$, can be prepared, as shown in Equation F, by reacting sequentially a bis-carbamyl fluoride of Formula VIII with one mole equivalent of an oxime of Formula IX and one mole equivalent of an oxime of Formula X, in the presence of an acid acceptor.

EQUATION F

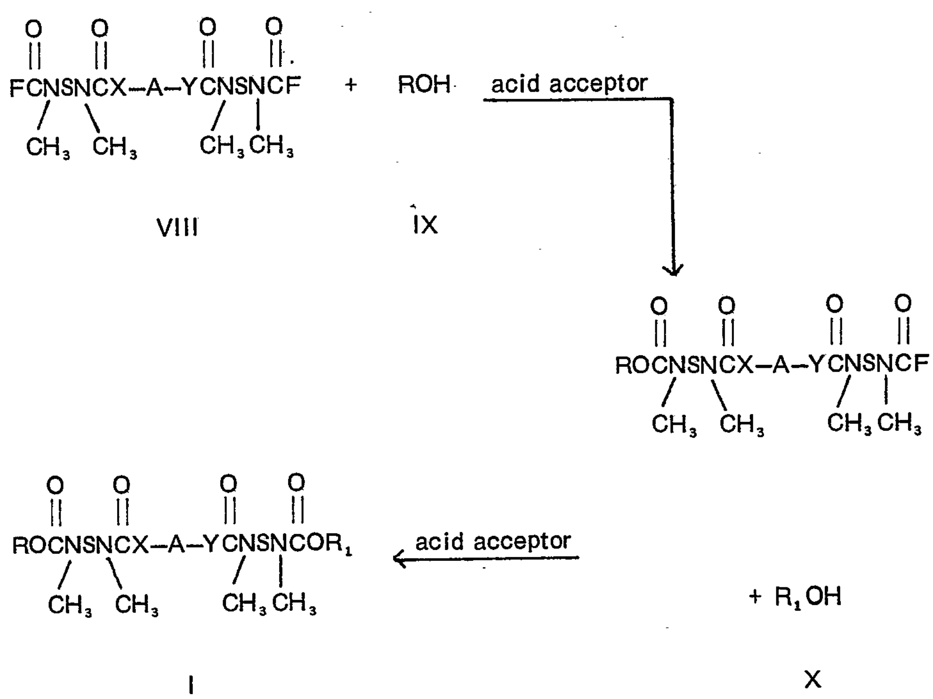


VIII IX I X

wherein R, $R_1$, X, Y, and A are as previously defined.

The reaction shown in Equation F can be performed in an inert organic solvent, such as benzene, toluene, the xylenes, methylene chloride, chloroform, ethylene dichloride, acetone, methyl ethyl ketone, dioxane, tetrahydrofuran, acetonitrile, or dimethylformamide. Mixtures of these solvents may be used. The reaction can be carried out at temperatures between 0° and 100°C, preferably between 20°—50°C. Pressure is not critical for the reaction procedure since pressures above and below atmospheric are suitable. For convenience, atmospheric pressure is preferred.

The acid acceptor used in Equation F can be tertiary organic amines, such as, trimethylamine, triethylamine, N,N-dimethylaniline, or pyridine, or inorganic bases, such as, the alkali metal or alkaline earth metal hydroxides, carbonates, or bicarbonates, and alkoxides, such as, sodium methoxide or potassium *tert*-butoxide.

The oximes of Formula IX and Formula X can be prepared by methods taught in U.S. 3,576,834, U.S. 3,530,220 and U.S. 3,217,037.

The bis-carbamyl fluorides of Formula VIII, used as reactants in Equation E and Equation F, can be prepared as shown in Equation G, by reacting a carbamyl fluoride of Formula XI with two mole equivalents of a compound of Formula VI, in the presence of an acid acceptor.

EQUATION G

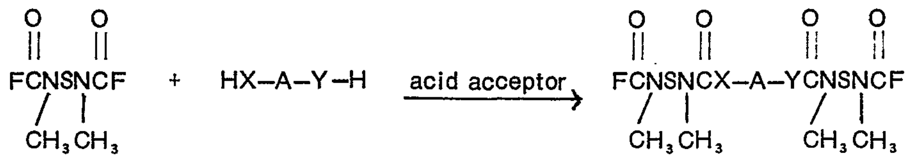


XI VI VIII

wherein X, Y, and A are as previously defined.

The reaction shown in Equation G can be carried out in an inert organic solvent, such as benzene, toluene, the xylenes, methylene chloride, chloroform, ethylene dichloride, acetone, methyl ethyl ketone, dioxane, tetrahydrofuran, ethyl acetate, acetonitrile, or dimethyl formamide. Mixtures of these solvents may be used. The reaction can be carried out at temperatures between 0° and 100°C, preferably between 20° and 80°C.

Pressure is not critical for the reaction procedure since pressures above and below atmospheric are suitable. For convenience, atmospheric is preferred.

The acid acceptor used in Equation G can be a tertiary organic amine, such as, trimethylamine, triethylamine, N,N-dimethylaniline, or pyridine, or an inorganic base, such as, the alkali metal and alkaline earth metal hydroxides, carbonates, bicarbonates, and alkoxides, such as, sodium methoxide and potassium *tert*-butoxide.

The carbamyl fluoride of Formula XI can be prepared by the methods taught in DT 2,654,246 and BE 848,914.

In the following examples all parts and percentages are by weight and temperatures in degrees centigrade unless otherwise specified. Examples 6 and 8 show the preparation of intermediates.

Example 1

Dimethyl N,N′-[[1,3-propanediylbis[oxycarbonyl(N-methylimino)thio(N-methylimino)carbonyloxy]]] bis-[ethanimidothioate]

A suspension of 4.7 g of N-methyl carbamic acid, diester with 1,3-propanediol, in 50 ml methylene chloride was cooled to .0°C and 5.2 g of sulfur dichloride was added. To this mixture was added dropwise a solution of 5.0 g of triethylamine in 20 ml methylene chloride, maintaining the reaction temperature between 0° and 5°C. When addition was complete, the yellow suspension was stirred 1 hr. at 0° to 5°C. A solution of 8.1 g of methyl N-[[(methylamino)carbonyl]oxy]ethanimidothioate and 4.9 g pyridine in 50 ml methylene chloride was added dropwise, maintaining the temperature between 0° and 5°C. When addition was complete, the reaction mixture was allowed to warm to ambient temperature and stirred overnight. The reaction mixture was washed with three 300 ml portions of water, a 200 ml portion of saturated sodium chloride solution, and dried over anhydrous magnesium sulfate. Distillation of the solvent under reduced pressure gave a brown, viscous oil. The reaction product was dissolved in 100 ml of 1-chlorobutane, and the organic solution was washed with three 300 ml portions of water, a small portion of saturated sodium chloride solution, and dried over anhydrous magnesium sulfate. Distillation of the solvent under reduced pressure afforded a brown, viscous syrup. The title compound was isolated from the crude reaction product by column chromatography on silica gel, eluting the product with ethyl acetate. By employing this procedure, there was obtained 2.4 g of substantially pure dimethyl N,N′-[[1,3-propanediyl-bis[oxycarbonyl(N-methylimino)thio(N-methylimino)carbonyloxy]]]bis-[ethanimidothioate] as a pale yellow glass.

| | | | | |
|---|---|---|---|---|
| Calcd for $C_{17}H_{30}N_6O_8S_4$: | C, 35.53; | H, 5.26; | N, 14.62; | S, 22.32 |
| Found: | C, 34.5; | H, 3.93; | N, 13.9; | S, 22.7 |
| | C, 34.6; | H, 4.52; | N, 13.8; | S, 22.7 |

Example 2

Dimethyl N,N′-[[1,2-ethanediylbis[oxycarbonyl(N-methylimino)thio(N-methylimino)carbonyloxy]]] bis-[ethanimidothioate]

A suspension of 4.4 g of N-methyl carbamic acid, diester with 1,2-ethanediol, in 50 ml methylene chloride was cooled to 0°C, and 5.2 g of sulfur dichloride was added. To this mixture was added dropwise a solution of 5.0 g of triethylamine in 20 ml $CH_2Cl_2$, maintaining the temperature between −5° and 0°C. When addition was complete, the yellow suspension was stirred at 0°C for 1 hr. A

solution of 8.1 g of methyl N-[[(methylamino)carbonyl]oxy]-ethanimidothioate and 4.9 g of pyridine in 25 ml methylene chloride was added dropwise at 0°C. When addition was complete, the reaction mixture was allowed to warm to ambient temperature and stirred 66 hr. The reaction mixture was washed with three 300 ml portions of water and dried over anhydrous magnesium sulfate. Distillation of the solvent at reduced pressure gave a brown viscous syrup. The reaction product was dissolved in 50 ml toluene; on addition of small amounts of ether with scratching, there was obtained 1.6 g of a tan solid, mp 117°—140°C. The title compound was isolated from the crude reaction product by column chromatography on silica gel, eluting the product with ethyl acetate. Employing this procedure, there was obtained 0.3 g of dimethyl-N,N'-[[1,2-ethanediylbis[oxycarbonyl(N-methylimino)thio(N-methylimino)carbonyloxy]]]bis-[ethanimidothioate], mp 150°—155°C.

Example 3

Methyl 2-(dimethylamino)-N-[[[[[[N-methyl[[[[[N-methyl[[[[3-[[[N-methyl[[N-methyl[N-(1-methylthioethylidene)aminooxycarbonyl]aminothio]]aminocarbonyloxy]]]propoxycarbonyl]]]] aminothio]]]]]aminocarbonyloxy]]]]]]-2-oxo-[ethanimidothioate]

A suspension of 4.7 g of N-methylcarbamic acid, diester with 1,3-propanediol, in 50 ml methylene chloride was cooled to 0°C, and 5.2 g of sulfur dichloride was added. To this mixture was added dropwise a solution of 5.0 g of triethylamine in 20 ml methylene chloride, maintaining the reaction temperature between 0° and 5°C. When addition was complete, the yellow suspension was stirred 1 hr. at 0° to 5°C. A solution of 4.0 g of methyl N-[[(methylamino) carbonyl] oxy]-ethanimidothioate, 5.5 g of methyl 2-(dimethylamino)-N-[[(methylamino)carbonyl]oxy]-2-oxo-ethanimidothioate, and 4.9 g of pyridine in 50 ml methylene chloride was added dropwise maintaining the temperature between 0° and 5°C. When addition was complete, the reaction mixture was allowed to warm to ambient temperature and stirred overnight. The reaction mixture was washed with three 300 ml portions of water and a 200 ml portion of a saturated solution of sodium chloride. The organic solution was dried over anhydrous magnesium sulfate, and the solvent was distilled under reduced pressure. The title compound can be isolated from the crude reaction product by a suitable procedure, such as, high pressure liquid chromatography.

Example 4

Dimethyl N,N'-[[1,2-ethanediylbis[aminocarbonyl(N-methylimino)thio(N-methyliminocarbonyloxy]]] bis-[ethanimidothioate]

A solution of 0.3 g of 1,2-ethanediamine and 1.0 g of triethylamine in 10 ml of tetrahydrofuran was added dropwise at ambient temperature to a solution of 2.7 g of methyl N-[[(N-[(N-fluoro-carbonyl-N-methylamino)thio-N-methylaminocarbonyloxy]]ethanimidothioate in 40 ml tetrahydrofuran; an exotherm of 5°C occurred. When addition was complete, the turbid reaction mixture was stirred an additional one hour. The resulting white solid was collected, washed with two 50 ml portions of water, a 50 ml portion of ethanol, and dried to afford 1.4 g of dimethyl N,N' - [[1,2 - ethanediylbis[aminocarbonyl(N - methylimino)thio(N - methylimino)carbonyloxy]]]bis - [ethanimidothioate], mp 166—168°C.

| | | | | |
|---|---|---|---|---|
| Calcd for $C_{16}H_{30}N_8O_6S_4$: | C, 34.52; | H, 5.07; | N, 20.13; | S, 23.04 |
| Found: | C, 34.7; | H, 5.58; | N, 20.0; | S, 21.3 |
| | C, 34.3; | H, 5.61; | N, 20.1; | S, 21.1 |

Example 5

Dimethyl N,N'-[[1,4-phenylenebis[oxycarbonyl(N-methylimino)thio(N-methylimino)carbonyloxy]]] bis-[ethanimidothioate]

A solution of 1.0 g of triethylamine in 10 ml tetrahydrofuran was added dropwise at ambient temperature to a solution of 2.7 g of methyl N-[[(N-[(N-fluorocarbonyl-N-methylamino)thio-N-methyl-amino]carbonyloxy]]-ethanimidothioate and 0.55 g of 1,4-benzenediol in 25 ml tetrahydrofuran. The reaction solution was stirred 2 hr. at ambient temperature, heated at reflux for 2 hr., then stirred at ambient temperature for an additional 86 hr. The resulting solid was collected, washed with a small volume of tetrahydrofuran-ether (3:2 v/v), and dried to give 1.8 g of dimethyl N,N' - [[1,4 - phenylene - bis - [oxocarbonyl(N - methylimino)thio(N - methylimino) - carbonyloxy]]]bis - [ethanimidothioate], m.p. 175—178°C.

| | | | | |
|---|---|---|---|---|
| Calcd for $C_{20}H_{28}N_6O_3S_4$: | C, 39.46; | H, 4.63; | N, 13.80; | S, 21.07 |
| Found: | C, 39.2; | H, 4.71; | N, 14.3; | S, 20.2 |

Example 6

N,N'-[[1,3-Propanediylbis[oxycarbonyl(N-methylimino)thio(N-methylimino)]]bis-[carbamic fluoride]

To a solution of 7.0 g of N,N'-thiobis[N-methyl carbamic fluoride] and 1.44 g of 1,3-propane diol in 100 ml of acetonitrile was added dropwise at ambient temperature 4.0 g of triethylamine. When addition was complete, a slow exotherm of 6°C occurred. The reaction mixture was stirred at ambient

temperature for 4 hrs. then heated at 80°C for 0.5 hr. The yellow reaction solution was cooled to ambient temperature, and the solvent was distilled under reduced pressure. The crude reaction product was dissolved in 100 ml of methylene chloride and the organic solution was washed with two 100 ml portions of water followed by a 100 ml portion of saturated sodium chloride solution. The organic solution was dried over anhydrous magnesium sulfate, and the solvent was distilled under reduced pressure to 6.4 g of substantially pure N,N' - [[1,3 - propanediylbis[oxocarbonyl - (N - methylimino)-thio(N - methylimino)]]]bis - [carbamic - fluoride] as a yellow-brown oil.

Calcd for $C_{11}H_{18}F_2N_4O_6S_2$: C, 32.67; H, 4.49; N, 13.85; S, 15.86
Found: C, 32.2; H, 4.37; N, 13.9; S, 16.0

Example 7

Dimethyl N,N'-[[1,3-propanediylbis[oxycarbonyl(N-methylimino)thio(N-methylimino)carbonyloxy]]] bis-[ethanimidothioate]

To a solution of 6.0 g of N,N'-[[1,3-propanediylbis-[oxycarbonyl(N-methylimino)thio(N-methylimino)]]]bis-[carbamic fluoride] and 3.3 g of methyl N-hydroxyethanimidothioate in 100 ml tetrahydrofuran was added dropwise at ambient temperature 3.3 g of triethylamine. When addition was complete, the reaction mixture was stirred at ambient temperature until thin-layer chromatography indicated that reaction was complete. The solvent was distilled under reduced pressure, and the residue was dissolved in 100 ml methylene chloride. The organic solution was washed with two 100 ml portions of water, and a 100 ml portion of saturated sodium chloride. The organic solution was dried over anhydrous magnesium sulfate, and the solvent was distilled under reduced pressure. The title compound can be isolated from the crude reaction product by an appropriate purification technique.

Example 8

Methyl N-[[[[[[N-methyl[[[[[N-methyl[[[[3-[[[N-methyl[[N-methyl[fluoro-carbonyl]aminothio]]-aminocarbonyloxy]]]-propoxy carbonyl]]]]aminothio]]]]]aminocarbonyloxy]]]]]]-ethanimidothioate

To a solution of 4.0 g of N,N'-[[1,3-propanediylbis[oxycarbonyl(N-methylimino)thio(N-methylimino)]]bis-[carbamicfluoride] and 1.0 g of methyl N-hydroxyethanimidothioate in 75 ml of tetrathydrofuran was added dropwise at ambient temperature 1.0 g of triethylamine. When addition was complete, the reaction mixture was stirred at ambient temperature until thin-layer chromatography indicated that reaction was complete. The solvent was distilled at reduced pressure, and the crude reaction product was dissolved in 100 ml methylene chloride. The organic solution was washed with two 100 ml portions of water followed by a 100 ml portion of saturated sodium chloride. The organic solution was dried over anhydrous magnesium sulfate, and the solvent was distilled under reduced pressure. The title compound can be isolated from the crude reaction product by an appropriate purification technique.

Example 9

Methyl 2-(dimethylamino)-N-[[[[[[N-methyl[[[[[N-methyl-[[[[3-[[[N-methyl[[N-methyl[N-(1-methylthioethylidene)-aminooxycarbonyl]aminothio]]aminocarbonyloxy]]]propoxycarbonyl]]]]-aminothio]]]]]aminocarbonyloxy]]]]]]-2-oxo-[ethanimidothioate]

To a solution of 4.9 g of methyl N - [[[[[[N - methyl[[[[[N - methyl[[[[3 - [[[N - methyl[[N - methyl[fluorocarbonyl]aminothio]]amino - carbonyloxy]]]propoxycarbonyl]]]]aminothio]]]]] - amino-carbonyloxy]]]]]]ethanimidothioate and 1.6 g of methyl 2 - (dimethylamino) - N - hydroxy - 2 - oxo - ethanimidothioate in 50 ml of tetrahydrofuran 5 ml of triethylamine was added dropwise at ambient temperature. When addition was complete, stirring was continued at ambient temperature until thin-layer chromatography indicated the reaction was complete. The solvent was distilled under reduced pressure, and the residue was dissolved in 100 ml of methylene chloride. The organic solution was washed with two 100 ml portions of water followed by a 100 ml portion of saturated sodium chloride solution. The organic solution was dried over anhydrous magnesium sulfate, and the solvent was distilled under reduced pressure. The title compounds can be isolated from the crude reaction product by an appropriate purification technique.

Example 10

Dimethyl N,N'-[[2,2-propanediylbis[4,1-phenyleneoxycarbonyl(N-methylimino)thio(N-methylimino)-carbonyloxy]]]bis[ethanimidothioate]

To a solution of 2.7 g of methyl N-[[[N-[[(N-fluorocarbonyl-N-methylimino)thio]]-N-methylimino-carbonyloxy]]]ethanimidothioate and 1.1 g of 4,4'-(2,2-propanediyl)bis(phenol) in 40 ml of tetrahydrofuran was added dropwise at ambient temperature 1.1 g of triethylamine. The reaction solution was stirred overnight at ambient temperature then refluxed 6 hrs. The reaction mixture was diluted with 100 ml of water, and the aqueous mixture was extracted with 100 ml of ethyl acetate. The organic phase was washed consecutively with 100 ml of 5% sodium hydroxide solution, two 100 ml portions of water, and 100 ml of saturated sodium chloride solution. The organic solution was dried over anhydrous magnesium sulfate, and the solvent was distilled under reduced pressure. The title

compound was isolated from the crude reaction product by column chromatography on silica gel. Elution of the column with a mixture of ethyl acetate-hexane (1:1, v/v) gave unreacted starting materials. Elution with ethyl acetate afforded the title compound as a colorless viscous oil. Trituration with ether gave a white solid which was suspended in a mixture of ether-hexane (1:1, v/v), collected, and dried to give 1.6 g of dimethyl N,N′ - [[2,2 - propanediyl - bis[4,1 - phenyleneoxycarbonyl(N - methylimino)thio(N - methylimino) - carbonyloxy]]bis[ethanimidothioate], m.p. 146°—150°C.

Calcd. for $C_{29}H_{38}N_6O_8S_4$: C, 47.92; H, 5.27; N, 11.56; S, 17.65
Found: C, 47.6; H, 5.32; N, 11.9; S, 17.0; S, 17.4

By employing the appropriate procedures as described in Examples 1—10, the compounds shown in Tables I—VIII can be prepared.

TABLE I

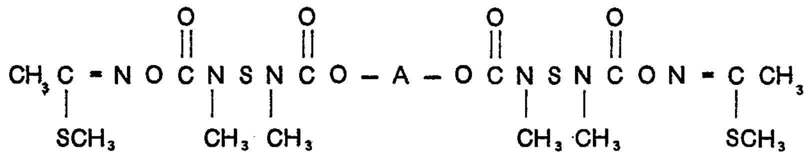

| A | Physical Property |
|---|---|
| 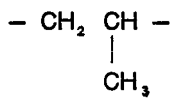 | C=O (5.82μ) |
| 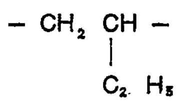 | |
| 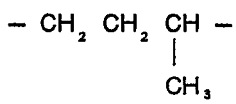 | |
| $-(CH_2)_4-$ | m.p. 148–150°C |
| $-(CH_2)_5-$ | |
| $-(CH_2)_8-$ | m.p. 118–122°C |
| $-(CH_2)_9$ | |
| 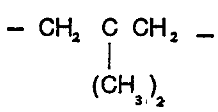 | C=O (5.83μ) |
| 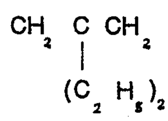 | C=O (5.84μ) |
| 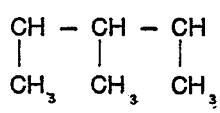 | C=O (5.84μ) |
| 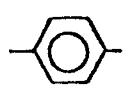 | m.p. 175–178°C |
| 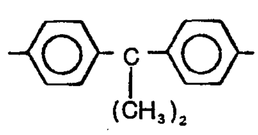 | m.p. 146–150°C |
| 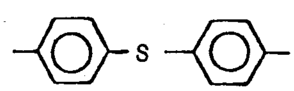 | C=O (5,81μ) |
| 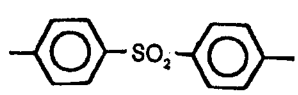 | C=O (5.80μ) |

TABLE I (Continued)

| A | Physical Property |
|---|---|
| 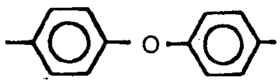 | |
| $(CH_2)_2-O-(CH_2)_2-$ | |
| 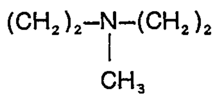 | |
| 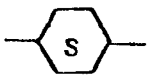 | |
| 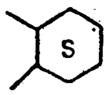 | |
| 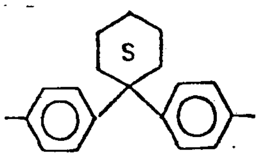 | C=O (5.80μ) |
| 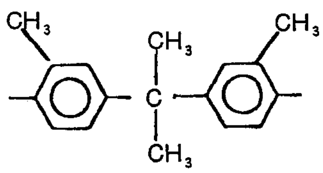 | C=O (5.80μ) |
| 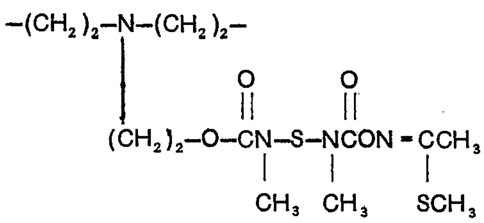 | C=O (5.82μ) |
| 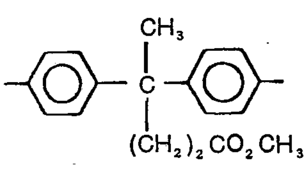 | |
| 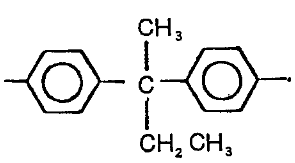 | |
| 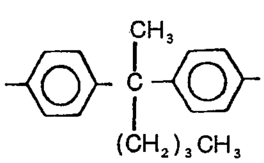 | |

TABLE I (Continued)

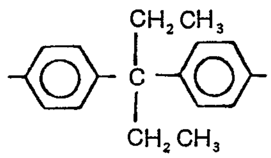

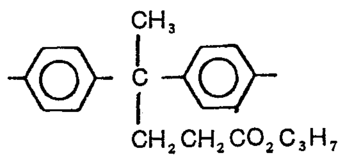

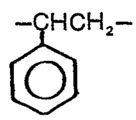

$CH_2CH_2OCH_2CH_2OCH_2CH_2$

$CH_2CH_2(OCH_2CH_2)_2OCH_2CH_2$

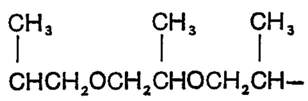

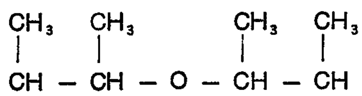

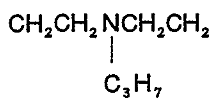

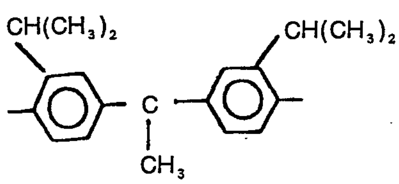

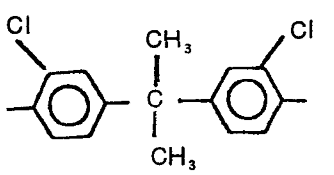

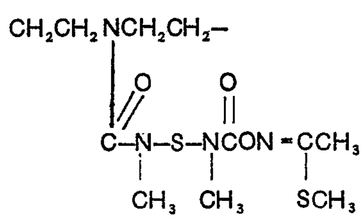

TABLE II

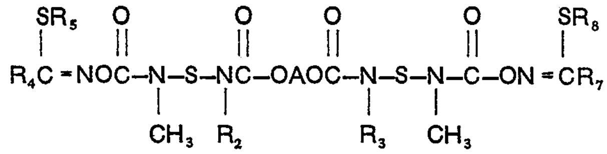

| $R_4$ | $R_7$ | $R_2$ | $R_3$ | $R_5$ | $R_8$ | A |
|---|---|---|---|---|---|---|
| $C_2H_5$ | $CH_3$ | $CH_3$ | $CH_3$ | $CH_3$ | $CH_3$ | $CH_2CH_2$ |
| $C_2H_5$ | $CH_3$ | $CH_3$ | $CH_3$ | $CH_3$ | $CH_3$ | $CH_2CH$<br>$CH_3$ |
| $C_2H_5$ | $CH_3$ | $CH_3$ | $CH_3$ | $CH_3$ | $CH_3$ | $(CH_2)_3$ |
| $C_2H_5$ | $CH_3$ | $CH_3$ | $CH_3$ | $CH_3$ | $CH_3$ | 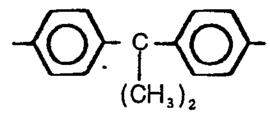 |
| $CH_3$ | $CH_3$ | $C_2H_5$ | $CH_3$ | $CH_3$ | $CH_3$ | $CH_2CH_2$ |
| $CH_3$ | $CH_3$ | $C_2H_5$ | $CH_3$ | $CH_3$ | $CH_3$ | $CH_2CH$<br>$CH_3$ |
| $CH_3$ | $CH_3$ | $CH_3$ | $CH_3$ | $C_2H_5$ | $CH_3$ | $(CH_2)_3$ |
| $CH_3$ | $CH_3$ | $CH_3$ | $CH_3$ | $C_2H_5$ | $CH_3$ | 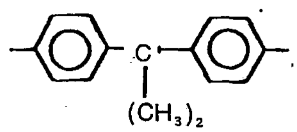 |
| $C_3H_7$ | $CH_3$ | $CH_3$ | $CH_3$ | $CH_3$ | $CH_3$ | $CH_2CH_2$ |
| $C_3H_7$ | $CH_3$ | $CH_3$ | $CH_3$ | $CH_3$ | $CH_3$ | $CH\ CH$<br>$CH_3$ |
| $C_3H_7$ | $CH_3$ | $CH_3$ | $CH_3$ | $CH_3$ | $CH_3$ | $(CH_2)_3$ |
| $C_3H_7$ | $CH_3$ | $CH_3$ | $CH_3$ | $CH_3$ | $CH_3$ | 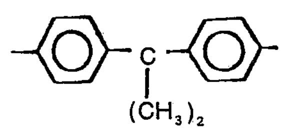 |
| $CH_3$ | $CH_3$ | $C_3H_7$ | $CH_3$ | $CH_3$ | $CH_3$ | $CH_2CH_2$ |
| $CH_3$ | $CH_3$ | $C_3H_7$ | $CH_3$ | $CH_3$ | $CH_3$ | $CH_2CH$<br>$CH_3$ |
| $C_2H_5$ | $C_2H_5$ | $CH_3$ | $CH_3$ | $CH_3$ | $CH_3$ | 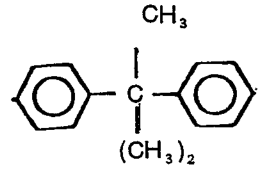 |
| $C_3H_7$ | $C_3H_7$ | $CH_3$ | $CH_3$ | $CH_3$ | $CH_3$ | 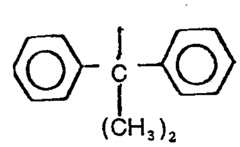 |

TABLE II (Continued)

| $R_4$ | $R_7$ | $R_2$ | $R_3$ | $R_5$ | $R_8$ | A |
|---|---|---|---|---|---|---|
| $C_2H_5$ | $C_3H_7$ | $CH_3$ | $CH_3$ | $CH_3$ | $CH_3$ | 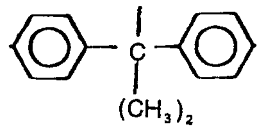 |
| $CH_3$ | $CH_3$ | $C_3H_7$ | $CH_3$ | $CH_3$ | $CH_3$ | $(CH_2)_3$ |
| $CH_3$ | $CH_3$ | $C_3H_7$ | $CH_3$ | $CH_3$ | $CH_3$ | 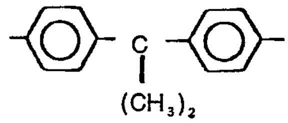 |
| $CH_3$ | $CH_3$ | $CH_3$ | $CH_3$ | $C_3H_7$ | $CH_3$ | $CH_2CH_2$ |
| $CH_3$ | $CH_3$ | $CH_3$ | $CH_3$ | $C_3H_7$ | $CH_3$ | $CH_2\underset{CH_3}{\underset{\vert}{C}}H$ |
| $CH_3$ | $CH_3$ | $CH_3$ | $CH_3$ | $C_3H_7$ | $CH_3$ | $(CH_2)_3$ |
| $CH_3$ | $CH_3$ | $CH_3$ | $CH_3$ | $C_3H_7$ | $CH_3$ | 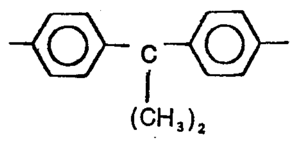 |
| $CH_3OCH_2$ | $CH_3$ | $CH_3$ | $CH_3$ | $CH_3$ | $CH_3$ | $CH_2CH_2$ |
| $CH_3OCH_2$ | $CH_3$ | $CH_3$ | $CH_3$ | $CH_3$ | $CH_3$ | $CH_2\underset{CH_3}{\underset{\vert}{C}}H$ |
| $CH_3OCH_2$ | $CH_3$ | $CH_3$ | $CH_3$ | $CH_3$ | $CH_3$ | $(CH_2)_3$ |
| $CH_3$ | $CH_3$ | $C_2H_5$ | $CH_3$ | $CH_3$ | $CH_3$ | $(CH_2)_3$ |
| $CH_3$ | $CH_3$ | $C_2H_5$ | $CH_3$ | $CH_3$ | $CH_3$ | 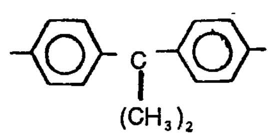 |
| $CH_3$ | $CH_3$ | $CH_3$ | $CH_3$ | $C_2H_5$ | $CH_3$ | $CH_2CH_2$ |
| $CH_3$ | $CH_3$ | $CH_3$ | $CH_3$ | $C_2H_5$ | $CH_3$ | $CH_2\underset{CH_3}{\underset{\vert}{C}}H$ |
| $CH_3OCH_2$ | $CH_3$ | $CH_3$ | $CH_3$ | $CH_3$ | $CH_3$ | $CH_2-\underset{(CH_3)_2}{\underset{\vert}{C}}-CH_2$ |
| $(CH_3)_2N\overset{O}{\overset{\Vert}{C}}$ | $CH_3$ | $CH_3$ | $CH_3$ | $CH_3$ | $CH_3$ | $CH_2CH_2$ |

TABLE II (Continued)

| $R_4$ | $R_7$ | $R_2$ | $R_3$ | $R_5$ | $R_8$ | A |
|---|---|---|---|---|---|---|
| $(CH_3)_2N\overset{O}{\overset{\|}{C}}$ | $CH_3$ | $CH_3$ | $CH_3$ | $CH_3$ | $CH_3$ | $CH_2\underset{CH_3}{\underset{\|}{CH}}$ |
| $(CH_3)_2N\overset{O}{\overset{\|}{C}}$ | $CH_3$ | $CH_3$ | $CH_3$ | $CH_3$ | $CH_3$ | $(CH_2)_3$ |
| $(CH_3)_2N\overset{O}{\overset{\|}{C}}$ | $CH_3$ | $CH_3$ | $CH_3$ | $CH_3$ | $CH_3$ | $CH_2-\underset{(CH_3)_2}{\underset{\|}{C}}-CH_2$ |
| $(CH_3)_2N\overset{O}{\overset{\|}{C}}$ | $(CH_3)_2N\overset{O}{\overset{\|}{C}}$ | $CH_3$ | $CH_3$ | $CH_3$ | $CH_3$ | $CH_2CH_2$ |
| $(CH_3)_2N\overset{O}{\overset{\|}{C}}$ | $(CH_3)_2N\overset{O}{\overset{\|}{C}}$ | $CH_3$ | $CH_3$ | $CH_3$ | $CH_3$ | $CH_2\underset{CH_3}{\underset{\|}{CH}}$ |
| $(CH_3)_2N\overset{O}{\overset{\|}{C}}$ | $(CH_3)_2N\overset{O}{\overset{\|}{C}}$ | $CH_3$ | $CH_3$ | $CH_3$ | $CH_3$ | $(CH_2)_3$ |
| $(CH_3)_2N\overset{O}{\overset{\|}{C}}$ | $(CH_3)_2N\overset{O}{\overset{\|}{C}}$ | $CH_3$ | $CH_3$ | $CH_3$ | $CH_3$ | 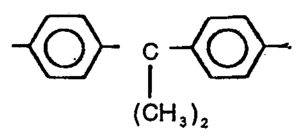 m.p. 111–115°C |

TABLE III

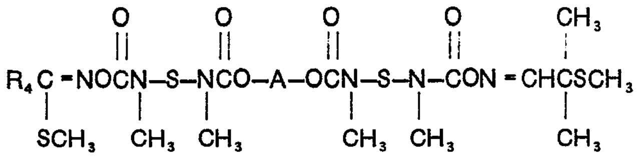

| $R_4$ | A |
|---|---|
| $CH_3$ | $CH_2CH_2$ |
| $CH_3$ | 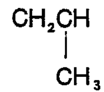 |
| $CH_3$ | $(CH_2)_3$ |
| $CH_3$ | 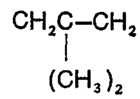 |
| $CH_3$ | 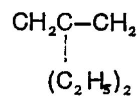 |
| $CH_3$ | 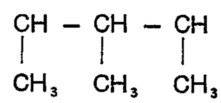 |
| $CH_3$ | 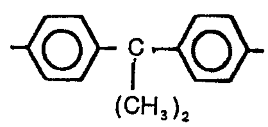 |
| 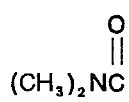 | $CH_2CH_2$ |
| 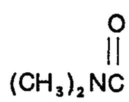 | 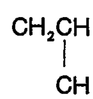 |
| 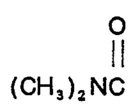 | $(CH_2)_3$ |
| 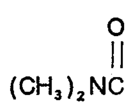 | 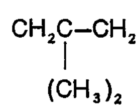 |
| 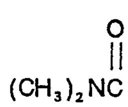 | 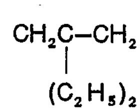 |

TABLE III (Continued)

| $R_4$ | A |
| --- | --- |
| 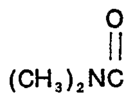 | 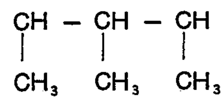 |
| 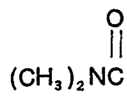 | 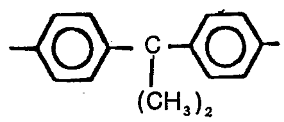 |

TABLE IV

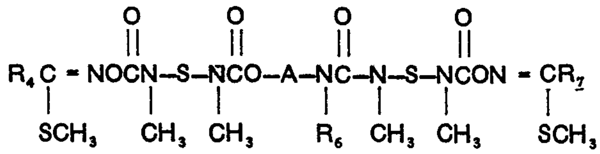

| $R_4$ | $R_7$ | $R_6$ | A | Physical Properties |
|---|---|---|---|---|
| $CH_3$ | $CH_3$ | H | $CH_2CH_2$ | |
| $CH_3$ | $CH_3$ | $CH_3$ | $CH_2CH_2$ | |
| $CH_3$ | $CH_3$ | $C_3H_7$ | $CH_2CH_2$ | |
| $CH_3$ | $CH_3$ | H | 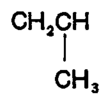 | |
| $CH_3$ | $CH_3$ | H | 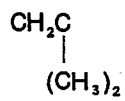 | m.p. 172–176°C |
| $CH_3$ | $CH_3$ | H | 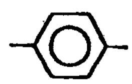 | |
| $CH_3$ | $CH_3$ | H | 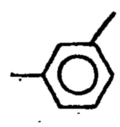 | |
| $CH_3$ | $CH_3$ | H | 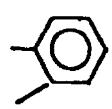 | |
| 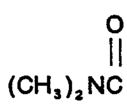 | $CH_3$ | H | $CH_2CH_2$ | |
| $CH_3$ | $CH_3$ | $CH_3$ | $-(CH_2)_3-$ | |
| 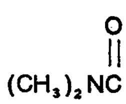 | $CH_3$ | $CH_3$ | $CH_2CH_2$ | |
| 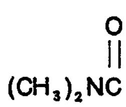 | $CH_3$ | $C_3H_7$ | $CH_2CH_2$ | |
| 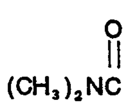 | $CH_3$ | H | 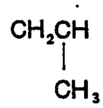 | |
| 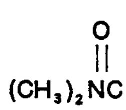 | $CH_3$ | H | 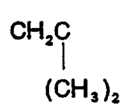 | |

TABLE IV (Continued)

| $R_4$ | $R_7$ | $R_6$ | A |
|---|---|---|---|
| $(CH_3)_2N\overset{\overset{O}{\|}}{C}$ | $CH_3$ | H | 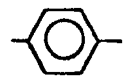 |
| $(CH_3)_2N\overset{\overset{O}{\|}}{C}$ | $CH_3$ | H | 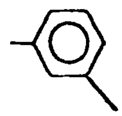 |
| $(CH_3)_2N\overset{\overset{O}{\|}}{C}$ | $CH_3$ | $CH_3$ | 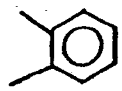 |
| $(CH_3)_2N\overset{\overset{O}{\|}}{C}$ | $(CH_3)_2N\overset{\overset{O}{\|}}{C}$ | H | $CH_2CH_2$ |
| $(CH_3)_2N\overset{\overset{O}{\|}}{C}$ | $(CH_3)_2N\overset{\overset{O}{\|}}{C}$ | $CH_3$ | $CH_2CH_2$ |
| $(CH_3)_2N\overset{\overset{O}{\|}}{C}$ | $(CH_3)_2N\overset{\overset{O}{\|}}{C}$ | $C_2H_5$ | $CH_2CH_2$ |
| $(CH_3)_2N\overset{\overset{O}{\|}}{C}$ | $(CH_3)_2N\overset{\overset{O}{\|}}{C}$ | H | $CH_2\underset{\underset{CH_3}{\|}}{CH}$ |
| $(CH_3)_2N\overset{\overset{O}{\|}}{C}$ | $(CH_3)_2N\overset{\overset{O}{\|}}{C}$ | H | $CH_2\underset{\underset{(CH_3)_2}{\|}}{CH}$ |
| $(CH_3)_2N\overset{\overset{O}{\|}}{C}$ | $(CH_3)_2N\overset{\overset{O}{\|}}{C}$ | H | 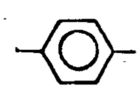 |
| $(CH_3)_2N\overset{\overset{O}{\|}}{C}$ | $(CH_3)_2N\overset{\overset{O}{\|}}{C}$ | H | 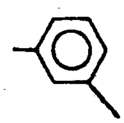 |
| $(CH_3)_2N\overset{\overset{O}{\|}}{C}$ | $(CH_3)_2N\overset{\overset{O}{\|}}{C}$ | H | 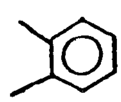 |

TABLE V

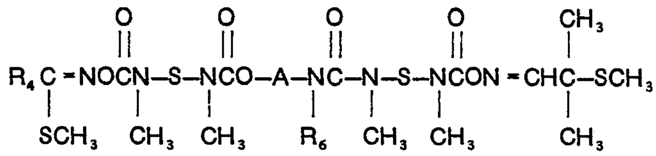

| $R_4$ | $R_6$ | A |
|---|---|---|
| $CH_3$ | H | $CH_2CH_2$ |
| $CH_3$ | $CH_3$ | $CH_2CH_2$ |
| $CH_3$ | $C_2H_5$ | $CH_2CH_2$ |
| $CH_3$ | H | 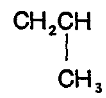 |
| $CH_3$ | H | 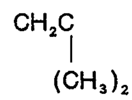 |
| $CH_3$ | H | 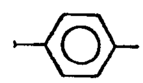 |
| $CH_3$ | H | 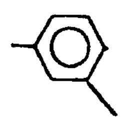 |
| $CH_3$ | H | 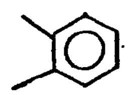 |
| 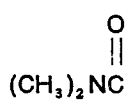 | H | $CH_2CH_2$ |
| 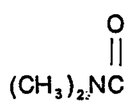 | $CH_3$ | $CH_2CH_2$ |
| 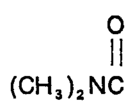 | $C_2H_5$ | $CH_2CH_2$ |
| 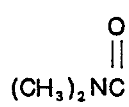 | H | 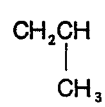 |
| 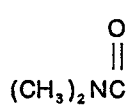 | H | 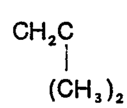 |

TABLE V (Continued)

| $R_4$ | $R_6$ | A |
|---|---|---|
| 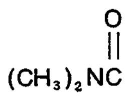 | H | 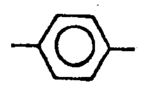 |
| 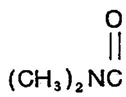 | H | 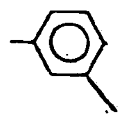 |
| 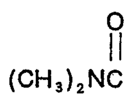 | H | 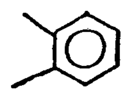 |

TABLE VI

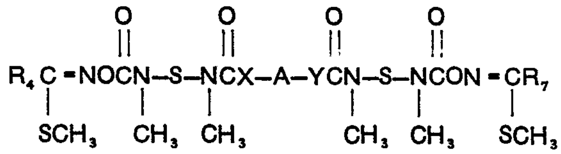

| $R_4$ | $R_7$ | X | Y | A | Physical Properties |
|---|---|---|---|---|---|
| $CH_3$ | $CH_3$ | NH | NH | $CH_2CH_2$ | m.p. 166–168°C |
| $CH_3$ | $CH_3$ | $NCH_3$ | $NCH_3$ | $CH_2CH_2$ | |
| $CH_3$ | $CH_3$ | NH | NH | $(CH_2)_3$ | |
| $CH_3$ | $CH_3$ | NH | NH | $(CH_2)_6$ | |
| $CH_3$ | $CH_3$ | $NCH_3$ | $NCH_3$ | 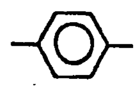 | |
| $CH_3$ | $CH_3$ | NH | NH | 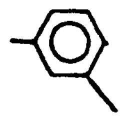 | |
| $CH_3$ | $CH_3$ | NH | NH | 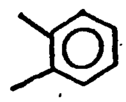 | |
| $CH_3$ | $CH_3$ | NH | NH | $CH_2CH$<br>\|<br>$CH_3$ | |
| $CH_3$ | $CH_3$ | NH | $NC_2H_5$ | $CH_2CH_2$ | |
| $CH_3$ | $CH_3$ | $NCH_3$ | NH | $(CH_2)_3$ | |
| $CH_3$ | $CH_3$ | NH | S | $CH_2CH_2$ | |
| $CH_3$ | $CH_3$ | NH | S | 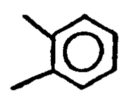 | |
| $CH_3$ | $CH_3$ | O | S | $CH_2CH_2$ | |
| $CH_3$ | $CH_3$ | S | S | $CH_2CH$<br>\|<br>$CH_3$ | |
| $CH_3$ | $CH_3$ | S | S | $(CH_2)_4$ | |
| O<br>‖<br>$(CH_3)_2NC$ | $CH_3$ | NH | NH | $CH_2CH_2$ | |
| O<br>‖<br>$(CH_3)_2NC$ | $CH_3$ | $NCH_3$ | $NCH_3$ | $CH_2CH_2$ | |
| O<br>‖<br>$(CH_3)_2NC$ | $CH_3$ | NH | NH | $(CH_2)_3$ | |

TABLE VI (Continued)

| $R_4$ | $R_7$ | X | Y | A | Physical Properties |
|---|---|---|---|---|---|
| $(CH_3)_2N\overset{\overset{O}{\|}}{C}$ | $CH_3$ | NH | NH | $(CH_2)_6$ | |
| $(CH_3)_2N\overset{\overset{O}{\|}}{C}$ | $CH_3$ | $NCH_3$ | $NCH_3$ | 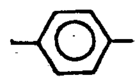 | |
| $(CH_3)_2N\overset{\overset{O}{\|}}{C}$ | $CH_3$ | NH | NH | 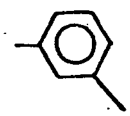 | |
| $(CH_3)_2N\overset{\overset{O}{\|}}{C}$ | $CH_3$ | NH | NH | 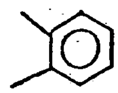 | |
| $(CH_3)_2N\overset{\overset{O}{\|}}{C}$ | $CH_3$ | NH | NH | $CH_2\underset{\underset{CH_3}{\|}}{CH}$ | |
| $(CH_3)_2N\overset{\overset{O}{\|}}{C}$ | $CH_3$ | NH | $NC_2H_5$ | $CH_2CH_2$ | |
| $(CH_3)_2N\overset{\overset{O}{\|}}{C}$ | $CH_3$ | NH | $NCH_3$ | $(CH_2)_3$ | |
| $(CH_3)_2N\overset{\overset{O}{\|}}{C}$ | $CH_3$ | NH | S | $CH_2CH_2$ | |
| $(CH_3)_2N\overset{\overset{O}{\|}}{C}$ | $CH_3$ | NH | S | 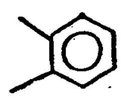 | |
| $(CH_3)_2N\overset{\overset{O}{\|}}{C}$ | $CH_3$ | S | S | $CH_2CH_2$ | |
| $(CH_3)_2N\overset{\overset{O}{\|}}{C}$ | $CH_3$ | S | S | $CH_2\underset{\underset{CH_3}{\|}}{CH}$ | |
| $(CH_3)_2N\overset{\overset{O}{\|}}{C}$ | $CH_3$ | S | S | $(CH_2)_4$ | |
| $(CH_3)_2N\overset{\overset{O}{\|}}{C}$ | $CH_3$ | O | S | $CH_2CH_2$ | |

TABLE VI (Continued)

| $R_4$ | $R_7$ | X | Y | A | Physical Properties |
|---|---|---|---|---|---|
| $(CH_3)_2N\overset{O}{\overset{\|}{C}}$ | $(CH_3)_2N\overset{O}{\overset{\|}{C}}$ | NH | NH | $CH_2CH_2$ | |
| $(CH_3)_2N\overset{O}{\overset{\|}{C}}$ | $(CH_3)_2N\overset{O}{\overset{\|}{C}}$ | $NCH_3$ | $NCH_3$ | $CH_2CH_2$ | |
| $(CH_3)_2N\overset{O}{\overset{\|}{C}}$ | $(CH_3)_2N\overset{O}{\overset{\|}{C}}$ | NH | NH | $(CH_2)_3$ | |
| $(CH_3)_2N\overset{O}{\overset{\|}{C}}$ | $(CH_3)_2N\overset{O}{\overset{\|}{C}}$ | NH | NH | $(CH_2)_6$ | |
| $(CH_3)_2N\overset{O}{\overset{\|}{C}}$ | $(CH_3)_2N\overset{O}{\overset{\|}{C}}$ | $NCH_3$ | $NCH_3$ | 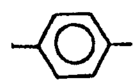 | |
| $(CH_3)_2N\overset{O}{\overset{\|}{C}}$ | $(CH_3)_2N\overset{O}{\overset{\|}{C}}$ | NH | NH | 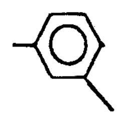 | |
| $(CH_3)_2N\overset{O}{\overset{\|}{C}}$ | $(CH_3)_2N\overset{O}{\overset{\|}{C}}$ | NH | NH | 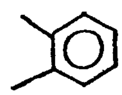 | |
| $(CH_3)_2N\overset{O}{\overset{\|}{C}}$ | $(CH_3)_2N\overset{O}{\overset{\|}{C}}$ | NH | NH | $CH_2\underset{CH_3}{\underset{\|}{C}}H$ | |
| $(CH_3)_2N\overset{O}{\overset{\|}{C}}$ | $(CH_3)_2N\overset{O}{\overset{\|}{C}}$ | NH | $NC_2H_5$ | $CH_2CH_2$ | |
| $(CH_3)_2N\overset{O}{\overset{\|}{C}}$ | $(CH_3)_2N\overset{O}{\overset{\|}{C}}$ | NH | $NCH_3$ | $(CH_2)_3$ | |
| $(CH_3)_2N\overset{O}{\overset{\|}{C}}$ | $(CH_3)_2N\overset{O}{\overset{\|}{C}}$ | NH | S | $CH_2CH_2$ | |
| $(CH_3)_2N\overset{O}{\overset{\|}{C}}$ | $(CH_3)_2N\overset{O}{\overset{\|}{C}}$ | NH | S | 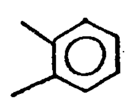 | |
| $(CH_3)_2N\overset{O}{\overset{\|}{C}}$ | $(CH_3)_2N\overset{O}{\overset{\|}{C}}$ | S | S | $CH_2CH_2$ | |

TABLE VI (Continued)

| $R_4$ | $R_7$ | X | Y | A | Physical Properties |
|---|---|---|---|---|---|
| $(CH_3)_2N\overset{O}{\overset{\|}{C}}$ | $(CH_3)_2N\overset{O}{\overset{\|}{C}}$ | S | S | $CH_2\underset{CH_3}{\underset{\|}{C}}H$ | |
| $(CH_3)_2N\overset{O}{\overset{\|}{C}}$ | $(CH_3)_2N\overset{O}{\overset{\|}{C}}$ | S | S | $(CH_2)_4$ | |
| $(CH_3)_2N\overset{O}{\overset{\|}{C}}$ | $(CH_3)_2N\overset{O}{\overset{\|}{C}}$ | O | S | $CH_2CH_2$ | |

TABLE VII

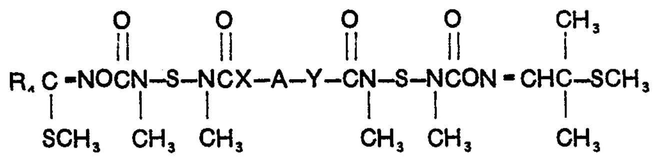

| $R_4$ | X | Y | A |
|---|---|---|---|
| $CH_3$ | NH | NH | $CH_2CH_2$ |
| $CH_3$ | $NCH_3$ | $NCH_3$ | $CH_2CH_2$ |
| $CH_3$ | NH | NH | $(CH_2)_3$ |
| $CH_3$ | NH | NH | 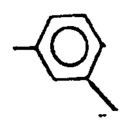 |
| $CH_3$ | NH | NH | 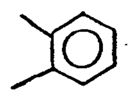 |
| $CH_3$ | NH | NH | $CH_2\underset{CH_3}{\underset{\mid}{CH}}$ |
| $CH_3$ | NH | $NC_2H_5$ | $CH_2CH_2$ |
| $CH_3$ | NH | S | $CH_2CH_2$ |
| $CH_3$ | NH | S | 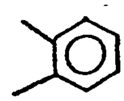 |
| $CH_3$ | O | S | $CH_2CH_2$ |
| $CH_3$ | S | S | $CH_2CH_2$ |
| $CH_3$ | S | S | $CH_2\underset{CH_3}{\underset{\mid}{CH}}$ |
| $CH_3$ | S | S | $(CH_2)_4$ |
| $(CH_3)_2N\overset{O}{\overset{\|}{C}}$ | NH | NH | $CH_2CH_2$ |
| $(CH_3)_2N\overset{O}{\overset{\|}{C}}$ | $NCH_3$ | $NCH_3$ | $CH_2CH_2$ |
| $(CH_3)_2N\overset{O}{\overset{\|}{C}}$ | NH | NH | $(CH_2)_3$ |
| $(CH_3)_2N\overset{O}{\overset{\|}{C}}$ | NH | NH | 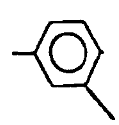 |

TABLE VII (Continued)

| $R_4$ | X | Y | A |
|---|---|---|---|
| 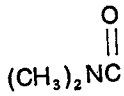 | NH | NH | 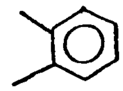 |
| 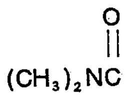 | NH | NH | 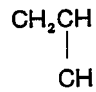 |
| 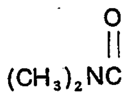 | NH | $NC_2H_5$ | $CH_2CH_2$ |
| 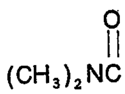 | NH | S | $CH_2CH_2$ |
| 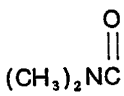 | NH | S | 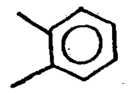 |
| 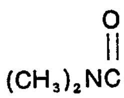 | O | S | $CH_2CH_2$ |
| 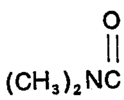 | S | S | $CH_2CH_2$ |
| 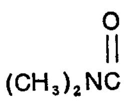 | S | S | 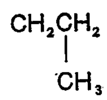 |
| 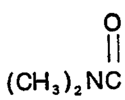 | S | S | $(CH_2)_4$ |

TABLE VIII

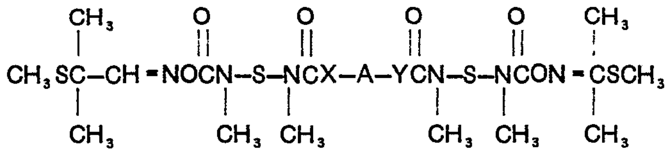

| X | A | Y |
|---|---|---|
| O | $CH_2CH_2$ | O |
| O | 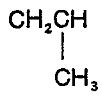 | O |
| O | $(CH_2)_3$ | |
| O | 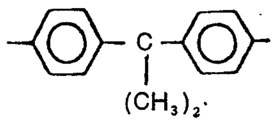 | O |
| O | $CH_2CH_2$ | NH |
| O | $CH_2CH_2$ | $NCH_3$ |
| O | 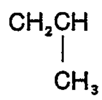 | NH |
| O | 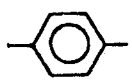 | NH |
| O | 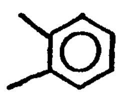 | NH |
| NH | $CH_2CH_2$ | NH |
| NH | 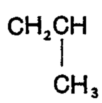 | NH |
| $NCH_3$ | $CH_2CH_2$ | $NCH_3$ |
| NH | 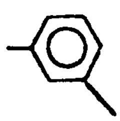 | NH |
| NH | 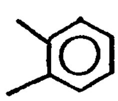 | NH |
| O | $CH_2CH_2$ | S |
| NH | $CH_2CH_2$ | S |
| S | $CH_2CH_2$ | S |
| S | $CH_2CH_2$ | S |

*Formulation*

Useful formulations of the compounds of Formula I can be prepared in conventional ways. They include e.g. dusts, granules, pellets, solutions, emulsions, wettable powders, and emulsifiable concentrates. Many of these may be applied directly. Sprayable formulations can be extended in suitable media and used at spray volumes of from a few litres to several thousand litres per hectare. High strength compositions are primarily used as intermediates for further formulations. The formulations, broadly, contain about 1% to 99% by weight of active ingredient(s) and at least one of (a) about 0.1% to 20% surfactant(s) and (b) about 1% to 99% solid or liquid diluent(s). More specifically, they will contain these ingredients in the following approximate percentages by weight:

| | Active Ingredient | Diluent(s) | Surfactant(s) |
|---|---|---|---|
| Wettable Powders | 20—90 | 0—74 | 1—10 |
| Oil Suspensions, Emulsions, Solutions (including Emulsifiable Concentrates) | 5—50 | 40—95 | 0—15 |
| Aqueous Suspensions | 10—50 | 40—84 | 1—20 |
| Dusts | 1—25 | 70—99 | 0—5 |
| Granules and Pellets | 1—95 | 5—99 | 0—15 |
| High Strength Compositions | 90—99 | 0—10 | 0—2 |

Lower or higher levels of active ingredient can, of course, be present depending on the intended use and the physical properties of the compound. For Example, many of the compounds are relatively low melting solids or glasses. In these cases, the strength of wettable powders and granules must be limited because of physical handling considerations. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd. Edn., Dorland Books, Caldwell, N.J. The more absorptive diluents are preferred for wettable powders and the denser ones for dusts. Typical liquid diluents and solvents are described in Marsden, "Solvents Guide", 2nd. Edn., Interscience, New York, 1950. Solubility under 0.1% is preferred for suspension concentrates; solution concentrates are preferably stable against phase separation at 0°C. McCutcheon's "Detergents and Emulsifiers Annual", MC Publishing Col, Ridgewood, New Jersey, as well as Sisely and Wood, "Encyclopedia of Surface Active Agents", Chemical Publ. Co., Inc., New York, 1964, list surfactant and recommended uses. All formulations can contain minor amounts of additives to reduce foam, caking, corrosion, microbiological growth, etc. Preferably, ingredients should be approved by the U.S. Environmental Protection Agency for the use intended.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", *Chemical Engineering*, Dec. 4, 1967, pp. 147ff. and "Perry's Chemical Engineer's Handbook", 4th. Edn., McGraw-Hill, N.Y., 1963, pp. *8*—59ff.

For further information regarding the art of formulation see for example:

J. B. Buchanan, U.S. Patent 3,576,834, April 27, 1971, Col. 5, Lines 36 through Col. 7, Line 70 and Ex. 1—4, 17, 106, 123—140;

R. R. Shaffer, U.S. Patent 3,560,616, Feb. 2, 1971, Col. 3, Line 48 through Col. 7, Line 26 and Examples 3—9, 11—18;

E. Somers, "Formulation", Chapter 6 in Torgeson, "Fungicides", Vol. I, Academic Press, New York, 1967.

Example A

| *Wettable Powder* | |
|---|---|
| Dimethyl N,N'-[[[2,2-propanediylbis[[4,1-phenyleneoxycarbonyl [N-methylimino]thio[N-methylimino]carbonyloxy]]]]]-bis[ethanimidothioate] | 30% |
| Dioctyl sodium sulfosuccinate | 1.5% |
| Sodium ligninsulfonate | 3% |
| Low viscosity methyl cellulose | 1.5% |
| Attapulgite | 64% |

The ingredients are thoroughly blended, passed through an air mill, to produce an average particle size under 15 microns, reblended, and sifted through a U.S.S. No. 50 sieve (0.3 mm opening) before packaging.

All compounds of the invention may be formulated in the same manner.

Percentages in this Example and in Examples B to H are on a weight basis.

Example B

| *Wettable Powder* | |
|---|---|
| Dimethyl N,N'-[[1,2-ethanediylbis[oxycarbonyl(N-methylimino) thio(N-methylimino)carbonyloxy]]]bis-[ethanimidothioate] | 50% |
| Sodium alkylnaphthalenesulfonate | 2% |
| Low viscosity methyl cellulose | 2% |
| Diatomaceous earth | 46% |

The ingredients are blended coarsely hammer-milled and then air milled to produce particles essentially all below 10 microns in diameter. The produce is reblended before packaging.

Example C

| *Dust* | |
|---|---|
| Wettable powder of Example B | 10% |
| Pyrophyllite (powder) | 90% |

The wettable powder and the pyrophyllite diluent are thoroughly blended and then packaged. The product is suitable for use as a dust.

Example D

| *Solution* | |
|---|---|
| Dimethyl N,N'-[[[2,2-propanediylbis[[4,1-phenyleneoxycarbonyl-[N-methylimino]thio[N-methylimino]carbonyloxy]]]]]-bis-[ethanimidothioate] | 30% |
| Dimethylformamide | 70% |

The ingredients are combined and stirred to produce a solution, which can be used for low volume applications.

Example E

| *Emulsifiable Concentrate* | |
|---|---|
| Dimethyl N-N'-[[1,3-propanediylbis-[oxycarbonyl(N-methylimino)-thio(N-methylimino)carbonyloxy]]]bis-[ethanimidothioate] | 30% |
| Blend of oil soluble sulfonates and polyoxyethylene ethers | 4% |
| Xylene | 60% |

The ingredients are combined and stirred with gentle warming to speed solution. A fine screen filter is included in packaging operation to insure the absence of any extraneous undissolved material in the product.

Example F

| *Granule* | |
|---|---|
| Wettable powder of Example A | 15% |
| Gypsum | 69% |
| Potassium sulfate | 16% |

The ingredients are blended in a rotating mixer and water sprayed on to accomplish granulation. When most of the material has reached the desired range of 1.0 to 0.42 mm. (U.S.S. No. 18 to 40 sieves), the granules are removed, dried, and screened. Over-size material is crushed to produce additional material in the desired range. These granules contain 4.5% active ingredient.

Example G

| *Extruded Pellet* | |
|---|---|
| Dimethyl N,N'-[[[2,2-propanediylbis[[4,1-phenyleneoxycarbonyl-[N-methylimino]thio[N-methylimino]carbonyloxy]]]]]-bis-[ethanimidothioate] | 25% |
| Anhydrous sodium sulfate | 10% |
| Crude calcium ligninsulfonate | 5% |
| Sodium alkylnaphthalenesulfonate | 1% |
| Calcium/magnesium bentonite | 59% |

The ingredients are blended, hammer milled and then moistened with about 12% water. The mixture is excluded as cylinders about 3 mm diameter which are cut to produce pellets about 3 mm long. These may be directly after drying, or the dried pellets may be crushed to pass a U.S.S. No. 20 sieve (0.84 mm opening). The granules held on a U.S.S. No. 40 sieve (0.42 mm opening) may be packaged for use and the fines recycled.

Example H

| *Aqueous Suspension* | |
|---|---|
| Dimethyl N,N′-[[1,2-ethanediylbis[oxycarbonyl(N-methylimino)-thio(N-methylimino)carbonyloxy]]]bis-[ethanimidothioate] | 25% |
| Hydrated attapulgite | 3% |
| Crude calcium ligninsulfonate | 10% |
| Sodium dihydrogen phosphate | 0.5% |
| Water | 61.5% |

The ingredients are ground together in a ball or roller mill until the solid particles have been reduced to diameters under 10 microns.

USE

The compounds of this invention are useful for control of insects and nematodes which are detrimental to agriculture.

As demonstrated in the following examples the compounds have provided a high degree of control of important agricultural pests with minimization of side effects known in prior art compounds such as phytotoxicity.

Only a slight effect has been noted on cotton with compounds within Formula I, i.e. dimethyl N,N - [[1,2 - ethanediylbis[oxycarbonyl - (N - methylimino)thio(N - methylimino)carbonyloxy]]] - bis - [ethanimidothioate]; dimethyl N,N′ - [[1,3 - propanediylbis[oxycarbonyl - (N - methylimino) - thio(N - methylimino)carbonyloxy]]] - bis - [ethanimidothioate]; and methyl 2 - (dimethylamino) - N - [[[[[[N - methyl - [[[[[N - methyl - [[[[3 - [[[N - methyl - [[N - methyl - [N - (1 - methylthioethylidene) - aminooxycarbonyl]aminothio]]aminocarbonyloxy]]]propoxycarbonyl]]]]aminothio]]]]]-aminocarbonyloxy]]]]]] - 2 - oxo - [ethanimidothioate].

The compounds readily control pestiferous insects belonging to such orders as Lepidoptera, Coleoptera, and Diptera. More specifically, insects controlled by the compounds of this invention include but are not limited to: southern armyworm (*Spodoptera eridania*), fall armyworm (*Spodoptera frugiperda*), soybean looper (*Pseudoplusia includens*), Mexican bean beetle (*Epilachna varivestis*), the house fly (*Musca domestica*), budworm (*Heliothis virescens*) and bollworm (*Heliothis zea*).

These compounds readily control pestiferous nematodes. More specifically nematodes controlled by compounds of this invention include, but are not limited to, the root-knot nematode, *Meloidogyne incognita*; lesion nematode, *Pratylenchus* spp. and dagger nematode, *Xiphinima*.

The insects are controlled by applying one or more of the compounds to the locus of infestation, to the area to be protected, or to the pests themselves. For the control of insects on agricultural crops, compounds of this invention are generally applied to the foliage or other plant parts which are infested or which are to be protected. Effective amounts to be applied depend upon the specific compound used, the species to be controlled, its life stage, its size and location, the amount of rainfall, the time of year, moisture, temperature, type of application plant spacing, and other variables. In general, .05 to 10 Kg/ha may be required for insect control in agriculture with rates of .15 to 5 Kg/ha usually being sufficient in many situations. In large scale field operations, rates in the range of .25 to 3 Kg/ha are generally used.

Nematodes are controlled by applying the compounds to the locus of infestation, to the area to be protected or to the pest themselves. For the control of nematodes in agricultural crops, a compound of this invention is generally applied to a portion of the plant or surrounding soil which is infested or which is to be protected. Effective amounts to be applied depend upon the species to be controlled, its life stage, its size and location, the amount of rainfall, the time of year, moisture, temperature, soil type, percentage of area treated, type of application, and other variables. In general, 3 to 30 Kg/ha may be required for nematode control in agriculture with rates of 5 to 10 Kg/ha usually being sufficient in many situations.

The compounds of this invention possess significant advantages over prior art compounds. For example, their improved residual insecticidal activity can reduce the need for closely spaced multiply sprays resulting in greater economy to the grower and dissemination of less insecticide in the environment. An additional advantage is the reduced side-effects on cotton. Treated leaves remain green and free of reddening.

The compounds of this invention can be mixed with fungicides, bactericides, acaricides, nematicides, insecticides, or other biologically active compounds in order to achieve desired results with a minimum expenditure of time, effort and material. Amounts of these biologically active materials

added for each part by weight of the composition of this invention may vary from 0.05 to 25 parts by weight. Suitable agents of this type are well-known to those skilled in the art. Some are listed below:

*Fungicides:*
methyl 2-benzimidazolecarbamate (carbendazim)
tetramethyl thiuram disulfide (thiuram)
*n*-dodecylguanidine acetate (dodine)
manganese ethylenebisdithiocarbamate (maneb)
1,4-dichloro-2,5-dimethoxybenzene (chloroneb)
methyl 1-(butylcarbamoyl)-2-benzimidazolecarbamate (benomyl)
2-cyano-N-ethylcarbamoyl-2-methoxyimino acetamide Curzate®
N-trichloromethylthiotetrahydrophthalimide (captan)
N-trichloromethylthiophthalimide (folpet)

*Bactericides:*
tribasic copper sulfate
streptomycin sulfate

*Acaricides:*
senecioic acid, ester with 2-*sec*-butyl-4,6-dinitrophenol ("Morocide")
6-methyl-1,3-dithiolo[2,3-B]quinonolin-2-one ("Morestan")
ethyl 4,4'(*p*-chlorophenyl)-2,2,2-trichloroethanol (Kelthane®)
bis(pentachloro-2,4-cyclopentadien-1-yl) (Pentac®)
tricyclohexyl tinhydroxide (Plictran®)

*Nematicides:*
2-[diethoxyphosphinylimino]-1,3-dithietane (Nematak®)
S-methyl 1-(dimethylcarbamoyl)-N-(methylcarbamoyloxy)thioformimidate
S-methyl 1-carbamoyl-N-(methylcarbamoyloxy)thioformidate
N-isopropylphosphoramidic acid, O-ethyl-O'[4-(methylthio)-*m*-tolyl]diester ("Nemacur")

*Insecticides:*
3-hydroxy-N-methylcrotonamide(dimethylphosphate)ester (Azodrin®)
methylcarbamic acid, ester with 2,3-dihydro-2,2-dimethyl-7-benzofuranol (Furandan®)
O-[2,4,5-trichloro-$\alpha$-(chloromethyl)benzyl]phosphoric acid, O',O'-dimethyl ester (Gardona®)
2-mercaptosuccinic acid, diethyl ester, S-ester with thionophosphoric acid, dimethyl ester (Malathion®)
phosphorothioic acid, O,O-dimethyl, O-*p*-nitrophenyl ester (methyl parathion)
methylcarbamic acid, ester with $\alpha$-naphthol (Sevin®)
methyl O-(methylcarbamoyl)thiolacetohydroxamate (methomyl)
N'-(4-chloro-*o*-tolyl)-N,N-dimethylformamidine (Galecron®)
O,O-diethyl-O-(2-isopropyl-4-methyl-6-pyrimidylphosphorothioate (Diazinon®)
octachlorocamphene (toxaphene)
O-ethyl O-*p*-nitrophenyl phenylphosphonothioate (EPN)
cyano(3-phenoxyphenyl)-methyl-4-chloro-$\alpha$-(1-methylethyl)-benzeneacetate (Pydrin®)
(3-phenoxyphenyl)methyl($\pm$)-*cis,trans*-3-(2,2-dichloroethenyl)-2,2-dimethylcyclopropanecarboxylate
dimethyl N,N'-[thiobis[(N-methylimino)carbonyloxy]]bis[ethanimidothioate] (Larvin®)
2-methyl-2-(methylthio)propionaldehyde O-methylcarbamoyloxime (Aldicarb)
O-ethyl-S-(*p*-chlorophenyl)ethylphosphonodithioate (Curacron®)
phosphorothiolothionic acid, O-ethyl-O-[4-(methylthio)-phenyl]-S-*n*-propyl ester (Bolstar®)

Example I

The foliage only of red kidney bean plants in the two-leaf stage is sprayed to run-off with dispersions of compounds of this invention at various concentrations. Dispersions are prepared by dissolving appropriately weighed quantities of the active ingredient in 10 ml of acetone and diluting to 100 ml of water containing surface active agent (Duponol® L—144 WDG) at 1:3000. After drying, leaves are excised and placed in covered 10 cm petri dishes along with moist filter paper to keep them fresh. Ten southern armyworm larvae are placed in each dish. The units are kept in a room maintained at 25 $\pm$ 2°C, 53 $\pm$ 5% RH. Results are recorded at the end of two days.

| Compound | Concentration % | % Mortality |
|---|---|---|
| dimethyl N,N-[(1,2-ethanediylbis[oxycarbonyl-(N-methylimino)thio(N-methylimino)carbonyloxy]]]-bis-[ethanimidothioate] | .01<br>.005 | 100<br>85 |
| dimethyl N,N′-[[1,3-propanediylbis[oxycarbonyl-(N-methylimino)thio(N-methylimino)carbonyloxy]]]-bis-[ethanimidothioate] | .01<br>.005 | 100<br>85 |
| dimethyl N,N′-[[1,5-pentanediylbis[oxycarbonyl-(N-methylimino)thio(N-methylimino)carbonyloxy]]]-bis-[ethanimidothioate] | .01<br>.005 | 95<br>85 |
| dimethyl N,N′-{2,2-propanediyl-bis-[4,1-phenyleneoxycarbonyl-(N-methylimino)thio-(N-methyl-imino)carbonyloxy]}bis(ethan-imidothioate) | .01<br>.005 | 100<br>100 |
| Untreated | — | 0 |

## Example II

The foliage of red kidney bean plants in the two-leaf stage infested with 20 soybean looper larvae (Wadmalaw strain)[1] is sprayed to run-off with dispersions of compounds of this invention at various concentrations. Dispersions are prepared by dissolving appropriately weighed quantities of the active ingredient in 5 ml of acetone and diluting to 100 ml of water containing surfactant (Trem® 014) at 1:3000. After drying, plants are placed under individual cylindrical cages, and watered daily to keep them fresh. The units are kept on the laboratory bench at room temperature. Results are recorded at the end of three days.

| Compound | Concentration % | % Mortality |
|---|---|---|
| dimethyl N,N′-[[1,3-propanediylbis[oxycarbonyl-(N-methylimino)thio(N-methylimino)carbonyloxy]]]-bis-[ethanimidothioate] | .12<br>.06 | 95<br>87 |
| dimethyl N,N′-[[1,5-pentanediylbis[oxycarbonyl-(N-methylimino)thio(N-methylimino)carbonyloxy]]]-bis-[ethanimidothioate] | .12<br>.06 | 90<br>80 |
| Untreated | — | 10 |

[1] Obtained at Wadmalaw Island, Georgia, during 1977.

Example III

The foliage of red kidney bean plants in the two-leaf stage is sprayed to run-off with dispersions of compounds of this invention at various concentrations. Dispersions are prepared by dissolving appropriately weighed quantities of the active ingredient in 10 ml of acetone and diluting to 100 ml with water containing surface active agent (Duponol® L—144 WDG) at 1:3000. After drying, plants are placed under artificial light in a room maintained at 25 ± 2°C, 54 ± 5% RH. After the designated period, leaves are excised and placed in covered 10 cm petri dishes along with moist filter paper to keep them fresh. Ten southern armyworm larvae are placed in each dish. The units are kept in a room maintained at 25 ± 2°C, 54 ± 5% RH. Results are recorded at the end of two days.

| | | Days (% Dead) | | |
|---|---|---|---|---|
| Compound | Concentration % | 2 | 7 | 9 |
| dimethyl-N,N-[[1,2-ethanediylbis[oxycarbonyl-(N-methylimino)thio(N-methylimino)carbonyloxy]]]-bis-[ethanimidothioate] | .01<br>.005 | 95<br>95 | 100<br>95 | 100<br>80 |
| dimethyl N-N'-[[1,3-propanediylbis[oxycarbonyl-(N-methylimino)thio(N-methylimino)carbonyloxy]]]-bis-[ethanimidothioate] | .01<br>.005 | 100<br>100 | 100<br>100 | 95<br>90 |
| dimethyl N,N-{2,2-propanediylbis[4,1-phenyleneoxy-carbonyl-(N-methylimino)-thio-(N-methylimino)carbonyloxy]}-bis(ethanimidothioate) | .01<br>.005 | 100<br>95 | 100<br>95 | —<br>— |
| Untreated Check | — | 0 | 0 | 0 |

## Example IV

Potted cotton plants approximately 25 cm high having 3—4 true leaves are sprayed to run-off with aqueous dispersions of compounds of this invention at 500 ppm. The sprays contain surface active agent (Duponol® L—144 WDG) at a concentration of 1:3000. Another set of plants is similarly treated with methomyl. After drying, plants are set out in the greenhouse and held for observation.

| Compound (500 ppm AI)[1)] | Rating[2)] (6 Days) |
|---|---|
| dimethyl-N,N-[[1,2-ethanediylbis[oxycarbonyl-(N-methylimino)thio(N-methylimino)carbonyloxy]]]-bis-[ethanimidothioate] | .2R |
| dimethyl-N-N'-[[1,3-propanediylbis[oxycarbonyl-(N-methylimino)thio(N-methylimino)carbonyloxy-bis-[ethanimidothioate] | .1R |
| methyl 2-(dimethylamino-N-[[[[[[N-methyl[[[[[N-methyl-[[[[3-[[[N-methyl[[N-methyl[N-(1-methylthioethylidene)-aminooxycarbonyl]aminothio]]-aminocarbonyloxy]]]propoxy-carbonyl]]]]aminothio]]]]]amino-carbonyloxy]]]]]]-2-oxo-[ethanimido-thioate] | Trace R |
| Methomyl (comparison treatment) | 3R |
| Untreated Control | 0 |

[1]AI = active ingredient.

[2]"R" denotes typical methomyl effect, i.e., reddening of older leaves, slight puckering and black stippling of younger leaves. Rating is on the basis of 0 to 10, with 10 indicating total leaf area involvement.

Example V

Fall armyworm larvae are treated topically with compounds of the invention. One microliter of each concentration used is applied to the dorso-thoracic area of each larva tested. The stock solutions are prepared by dissolving appropriately weighed quantities of active ingredient in predetermined quantities of acetone. Further diluting with acetone yields the desired concentrations. After treating the 15 fall armyworm larvae with each desired concentration, they are placed on artificial diet in 118 ml squat cups. The units are kept in a chamber at 27 ± 2°C. Results are recorded 2 days after treatment.

| Compound | Concentration (μg/larva) | % Mortality (48 hrs) |
|---|---|---|
| dimethyl-N,N-[[1,2-ethanediylbis[oxycarbonyl-(N-methylimino)thio(N-methylimino)carbonyloxy]]]-bis-[ethanimidothioate] | 4<br>2 | 93<br>87 |
| dimethyl N,N'-[[1,3-propanediylbis[oxycarbonyl-(N-methylimino)thio(N-methylimino)carbonyloxy]]]-bis-[ethanimidothioate] | 4<br>2 | 100<br>100 |
| dimethyl N,N'-[[1,5-pentanediylbis[oxycarbonyl-(N-methylimino)thio(N-methylimino)carbonyloxy]]]-bis-[ethanimidothioate] | 4<br>2 | 100<br>93 |
| Untreated | — | 13 |

Example VI

The under surfaces of four 4 cm red kidney bean leaf discs are treated with acetone solutions of compounds of this invention at various concentrations. Solutions are prepared by dissolving appropriately weighed quantities of the active ingredient in 10 ml of acetone and diluting further with the appropriate quantities of acetone. After drying, the discs are placed into covered 10 cm petri dishes along with moist filter paper to keep them fresh. Seven Mexican bean beetle larvae are placed into each dish. The units are kept in a room maintained at 25° ± 2°C, 50% RH. Results are recorded at the end of 5 days.

| Compound | Concentration % | % Mortality |
|---|---|---|
| dimethyl-N,N'-[[1,3-propanediylbis[oxycarbonyl-(N-methylimino)thio(N-methylimino)carbonyloxy]]]-bis-[ethanimidothioate] | .01 | 100 |
| dimethyl-N,N'-[[1,5-pentanediylbis[oxycarbonyl-(N-methylimino)thio(N-methylimino)carbonyloxy]]]-bis-[ethanimidothioate] | .01 | 84 |
| Untreated | — | 0 |

Example VII

Twenty-five house fly adults are secured in screened stainless steel ring cages and treated with acetone dispersions of compounds of this invention at various concentrations. The stock solution is prepared by dissolving appropriately weighed quantities of active ingredient in predetermined quantities of acetone. Further diluting with acetone yields the desired concentrations. After treating, the units are kept in a room maintained at 25° ± 2°C, 50% RH. Results are recorded at the end of 1 day.

| Compound | Concentration % | % Mortality |
|---|---|---|
| dimethyl-N-N'-[[1,3-propanediylbis[oxycarbonyl-(N-methylimino)thio(N-methylimino)carbonyloxy]]]-bis-[ethanimidothioate] | 0.2 | 100 |

Example VIII

Dimethyl N,N' - [[1,2 - ethanediylbis[oxycarbonyl(N - methylimino)thio(N - methylimino)-carbonyloxy]]] - bis - [ethanimidothioate], was dissolved in acetone and mixed into soil containing the root-knot nematode, *Meloidogyne incognita.* Dimethyl N,N' - [[1,3 - propanediylbis[oxycarbonyl(N - methylimino)thio - (N - methylimino)carbonyloxy]]]bis - [ethanimidothioate], was similarly dissolved in acetone and mixed into soil containing the root-knot nematode, *Meloidogyne incognita.* The treated soil samples were planted with cucumber seeds. After two weeks, the roots were examined for nematode injury and the results are summarized below.

| | kg/ha | % Nematode Control |
|---|---|---|
| dimethyl-N,N-[[1,2-ethanediylbis[oxycarbonyl-(N-methylimino)thio(N-methylimino)carbonyloxy]]]-bis-[ethanimidothioate] | 15 | 100 |
| dimethyl N-N'-[[1,3-propanediylbis[oxycarbonyl-(N-methylimino)thio(N-methylimino)carbonyloxy]]]-bis-[ethanimidothioate] | 15 | 100 |
| Untreated Control | | 0 |

"Consisting essentially off" is intended to have its customery meaning: namely, that all specified materials and conditions are very important in practicing the invention but that unspecified materials and conditions are not excluded as long as they do not prevent the benefits of the invention from being realized.

## Claims

1. A compound of the formula

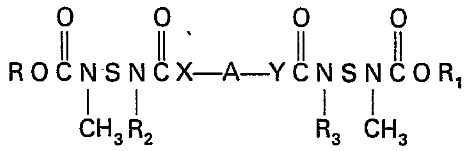

where R is 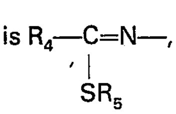, 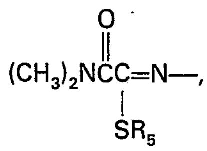, or 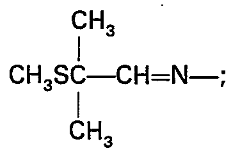; $R_1$ is 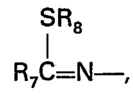, 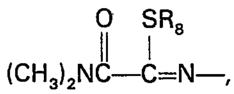, or 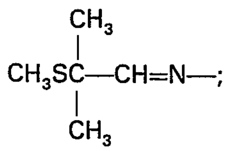;

$R_2$, $R_3$, $R_5$ and $R_8$ are independently $C_1$—$C_3$ alkyl;
$R_4$ and $R_7$ are independently $C_1$—$C_3$ alkyl or $CH_3OCH_2$—;

A is $—(CT_1T_2)_k(CT_3T_4)_l(CT_5T_6)_m$; 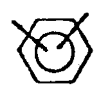 ; 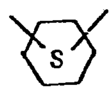 (in which S means saturated)

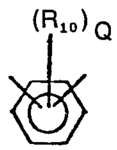 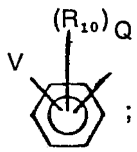 ; 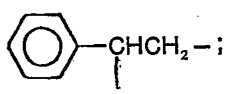 ; 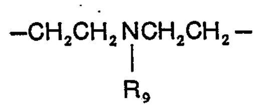 ;

or $-CT_1T_2CT_3T_4 \text{-[-}OCT_1T_2CT_3T_4\text{-]-}_p OCT_1T_2CT_3T_4-$

X and Y are independently $NR_6$, O or S;
$T_1$ to $T_6$ are independently hydrogen or $C_1$—$C_2$ alkyl;
$R_6$ is H or alkyl $C_1$—$C_3$;

$R_9$ is alkyl $C_1$—$C_3$, 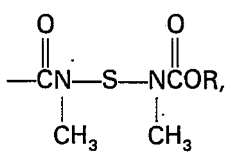, 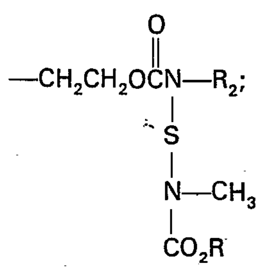;

V is O, $S(O)_n$, $CT_7T_8$;
$R_{10}$ is H, alkyl of $C_1$—$C_3$, Cl;
$T_7$ is H, $C_1$—$C_2$ alkyl, carboalkoxy alkyl of 3—8 carbon atoms, $C_2$—$C_4$ carboalkoxy;
$T_8$ is H, alkyl $C_1$—$C_4$;
$T_7$ and $T_8$ may be taken together to form a carbocyclic ring of 5—6 carbons;
Q is 0—2;
k, l, m are independently 0—3;
n, p are independently 0—2,

provided

a) the sum of k, l and m is at least two;
b) the total carbon content of $—(CT_1T_2)_k$; $(CT_3T_4)_l$ $(CT_5T_6)_m$— will not exceed g;

c) when A is 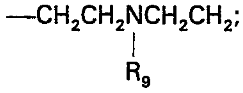

then X and Y are both oxygen and $R=R_1$;

d) when A is $CT_1T_2CT_3T_4-[OCT_1T_2CT_3Y_4]_p-OCT_1T_2CT_3T_4$, X and Y are both oxygen.

2. A compound of Claim 1 where $R = R_1$.

3. A compound of Claim 2 where R and $R_1$ both are

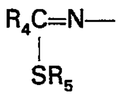 or 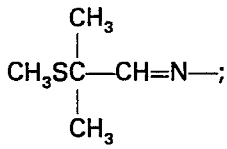; X and Y are both oxygen;

A is $(CT_1T_2)_k(CT_3T_4)_l(CT_3-T_6)_m$ or 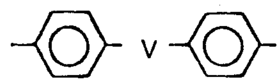; and

k, l and m are independently 0—3.

4. A compound of Claim 3 where R and $R_1$ both are 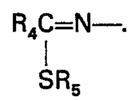.

5. A compound of claim 4 where $R_2$, $R_3$ and $R_5$ are methyl; $R_4$ is $C_1-C_2$ alkyl;

A is $(CT_1T_2)_k(CT_3T_4)_l(CT_3-T_6)_m$ or 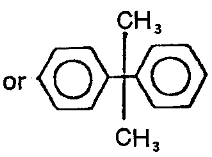; and $T_1-T_6$ are hydrogen;

provided the sum of k, l and m total not more than 5.

6. A compound of claim 1 which is dimethyl N,N′-[[1,2-ethanediylbis[oxycarbonyl(N-methylimino)thio(N-methylimino)carbonyloxy]]biz-[ethanimidothioate].

7. A compound of claim 1 which is dimethyl N-N′-[[1,3-propanediylbis[oxycarbonyl(N-methylimino)thio(N-methylimino)carbonyloxy]]bis-[ethanimidothioate].

8. A compound of claim 1 which is dimethyl N,N′-[[2,2-propanediylbis[4,1-phenyleneoxycarbonyl(N-methylimino)thio(N-methylimino)carbonyloxy]]bis-[ethanimidothioate].

9. An agricultural composition consisting essentially of a diluent, surfactant or mixtures thereof and a pesticide characterised in

that said pesticide is a compound of any of claims 1—8.

10. A method for controlling insects or nematodes by applying to a locus to be protected an insecticidally or nematicidally effective amount of a pesticide characterised in

that said pesticide is a compound of any of claims 1—8.

11. A method for preparing a compound of claim 1 which comprises

(a) where $R = R_1$, contacting in the presence of an acid acceptor

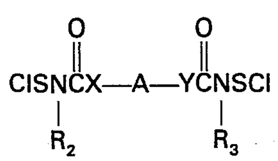 and 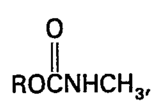, or

(b) contacting in the presence of an acid acceptor

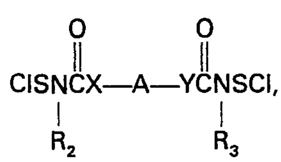, 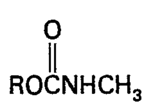 and 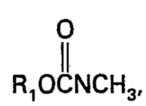, or

(c) where $R = R_1$ and $R_2 = R_3 = CH_3$, contacting in the presence of an acid acceptor

(i) HX—A—YH and 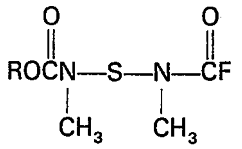 or

(ii) 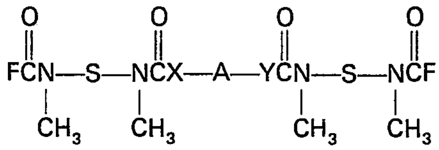 and ROH, or

(d) where R is different from $R_1$ and $R_2 = R_3 = CH_3$, contacting in the presence of an acid acceptor

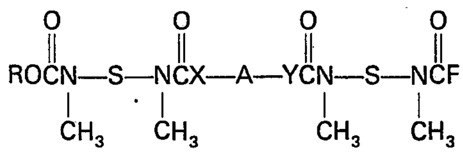 and $R_1OH$.

## Patentansprüche

1. Verbindung mit der Formel

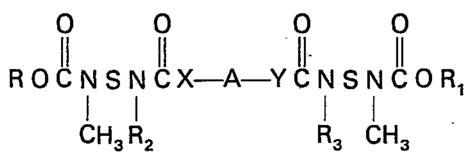

worin R die Bedeutung hat von 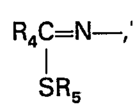, 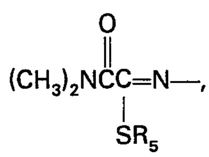, oder 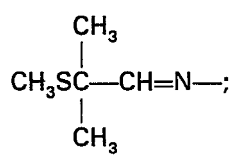;

$R_1$ die Bedeutung hat von 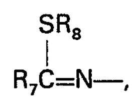, 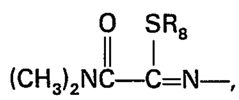, oder 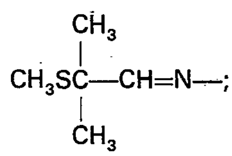;

$R_2$, $R_3$, $R_5$ und $R_8$ unabhängig $C_1$—$C_3$-Alkyl sind;
$R_4$ und $R_7$ unabhängig $C_1$—$C_3$-Alkyl oder $CH_3OCH_2$— sind;

A die Bedeutung hat von $-(CT_1T_2)_k(CT_3T_4)_l(CT_5T_6)_m$; 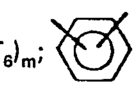; 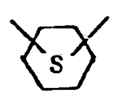

(worin S gesättigt bedeutet); 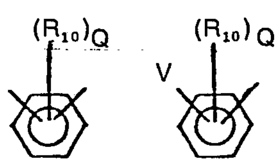 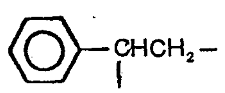; 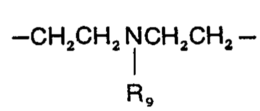;

oder $-CT_1T_2CT_3T_4-[-OCT_1T_2CT_3T_4-]_p OCT_1T_2CT_3T_4-$

X und Y unabhängig $NR_6$, O oder S sind;
$T_1$ bis $T_6$ unabhängig Wasserstoff oder $C_1$—$C_2$-Alkyl sind;
$R_6$ H oder Alkyl-$C_1$—$C_3$ ist;

$R_9$ die Bedeutung hat von Alkyl-$C_1$—$C_3$, 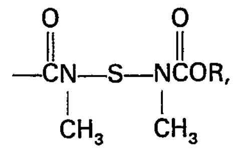 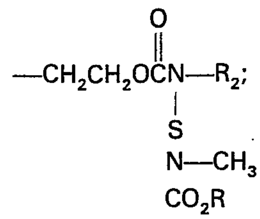

V die Bedeutung hat von O, $S(O)_n$, $CT_7T_8$;
$R_{10}$ H, Alkyl mit $C_1$—$C_3$, Cl ist;
$T_7$ H, $C_1$—$C_2$-Alkyl, Carboalkoxyalkyl mit 3 bis 8 Kohlenstoffatomen, $C_2$—$C_4$-Carboalkoxy ist;
$T_8$ H, Alkyl-$C_1$—$C_4$ ist;
$T_7$ und $T_8$ zusammengenommen werden können unter Bildung eines carbocyclischen Ringes mit 5 bis 6 Kohlenstoffen;
Q 0—2 ist;
k, l, m unabhängig 0—3 sind;
n, p unabhängig 0—2 sind;
vorausgesetzt, dass
a) die Summe von k, l und m mindestens 2 beträgt;
b) der Gesamtkohlenstoffgehalt von $—(CT_1T_2)_k; (CT_3T_4)_l\ (CT_5T_6)_m—$ 9 nicht überschreitet;
c) wenn A die Bedeutung hat von 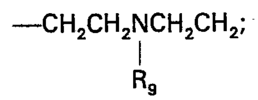
dann X und Y beide Sauerstoff sind und $R = R_1$;
d) wenn A die Bedeutung hat von $CT_1T_2CT_3T_4 -[OCT_1T_2CT_3Y_4]_p —OCT_1T_2CT_3T_4$, X und Y beide Sauerstoff sind.

2. Verbindung nach Anspruch 1, worin $R = R_1$.
3. Verbindung nach Anspruch 2, worin R und $R_1$ beide die Bedeutung haben von

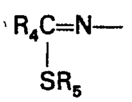 oder 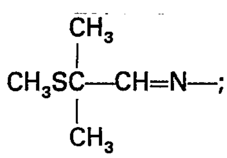 X und Y beide Sauerstoff sind;

A die Bedeutung hat von $(CT_1T_2)_k\ (CT_3T_4)_l\ (CT_3—T_6)_m$ oder 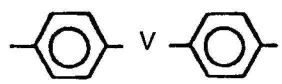

und k, l und m unabhängig 0—3 sind.
4. Verbindung nach Anspruch 3, worin R und $R_1$ beide

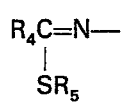 sind.

5. Verbindung nach Anspruch 4, worin $R_2$, $R_3$ und $R_5$ Methyl sind;
$R_4$ $C_1$—$C_2$-Alkyl ist;
A die Bedeutung hat von $(CT_1T_2)_k\ (CT_3T_4)_l\ (CT_3—T_6)_m$

oder 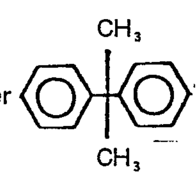 und $T_1$—$T_6$ Wasserstoff sind;

vorausgesetzt, dass die Summe von k, l und m insgesamt nicht mehr als 5 ist.
6. Verbindung nach Anspruch 1, nämlich Dimethyl-N,N'-[[1,2-Äthandiylbis[oxycarbonyl(N-methylimino)thio(N-methylimino)carbonyloxy]]]-bis-[äthanimidothioat].
7. Verbindung nach Anspruch 1, nämlich Dimethyl-N,N'-[[1,3-Propandiylbis[oxycarbonyl(N-methylimino)thio(N-methylimino)carbonyloxy]]]-bis-[äthanimidothioat].
8. Verbindung nach Anspruch 1, nämlich Dimethyl-N,N'-[[2,2-Propandiylbis[4,1-phenylenoxy-carbonyl(N-methylimino)thio(N-methylimino)-carbonyloxy]]]-bis-[äthanimidothioat].

9. Landwirtschaftliche Zusammensetzung, bestehend im wesentlichen aus einem Verdünnungsmittel, oberflächenaktiven Mittel oder Gemischen davon und einem Pestizid, dadurch gekennzeichnet, dass das Pestizid eine Verbindung nach einem beliebigen der Ansprüche 1—8 ist.

10. Verfahren zur Bekämpfung von Insekten oder Nematoden durch Auftrag auf einen zu schützenden Ort von einer insektizid oder nematizid wirksamen Menge eines Pestizids, dadurch gekennzeichnet, dass das Pestizid eine Verbindung nach einem beliebigen der Ansprüche 1 bis 8 ist.

11. Verfahren zur Herstellung einer Verbindung nach Anspruch 1 durch

(a) falls $R = R_1$, Kontakt in Anwesenheit eines Säureakzeptors von

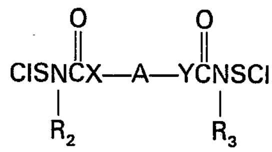 und 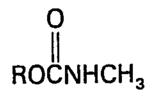 oder

(b) Kontakt in Anwesenheit eines Säureakzeptors von

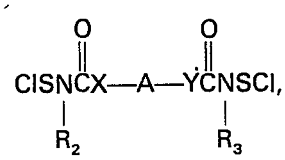, 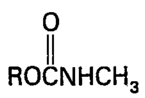 und 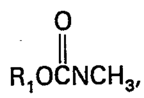, oder

(c) falls $R = R_1$ und $R_2 = R_3 = CH_3$, Kontakt in Anwesenheit eines Säureakzeptors von

(i) HX—A—YH und 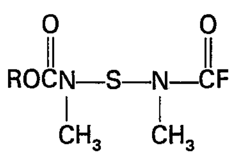 oder

(ii) 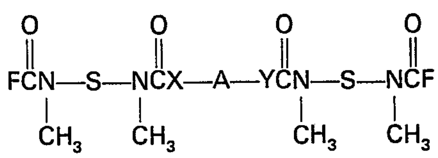 und ROH, oder

(d) falls R unterschiedlich von $R_1$ ist und $R_2 = R_3 = CH_3$, Kontakt in Anwesenheit eines Säureakzeptors von

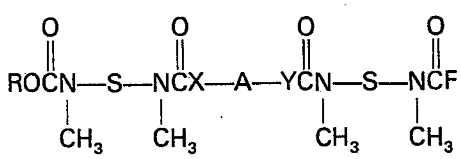 und $R_1OH$.

**Revendications**

1. Un composé de la formule

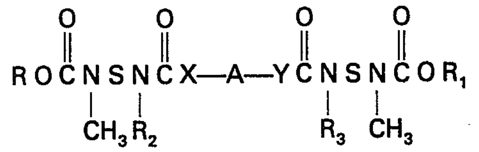

dans laquelle R est 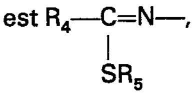, 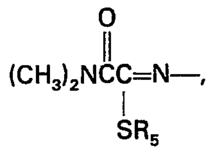, ou 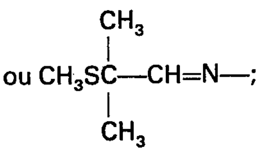;

$R_1$ est 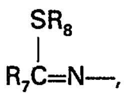, 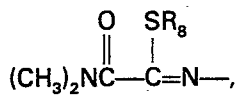, ou 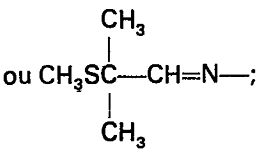;

$R_2$, $R_3$, $R_5$ et $R_8$ sont chacun indépendamment un groupe alcoyle en $C_1$—$C_3$;
$R_4$ et $R_7$ sont chacun indépendamment un groupe alcoyle en $C_1$—$C_3$ ou $CH_3OCH_2$—;

A est $-(CT_1T_2)_k(CT_3T_4)_l(CT_5T_6)_m$; 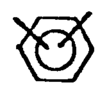; 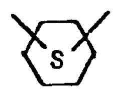 (où S veut dire "saturé");

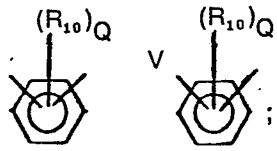; 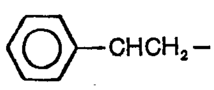; 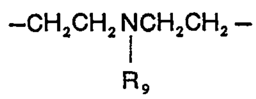;

ou $-CT_1T_2CT_3T_4 \left[ OCT_1T_2CT_3T_4 \right]_p OCT_1T_2CT_3T_4-$

X et Y sont chacun indépendamment $NR_6$, O ou S;
$T_1$ à $T_6$ sont chacun indépendamment l'hydrogène ou un groupe alcoyle en $C_1$—$C_2$;
$R_6$ est H ou un groupe alcoyle en $C_1$—$C_3$;

$R_9$ est un groupe alcoyle en $C_1$—$C_3$, 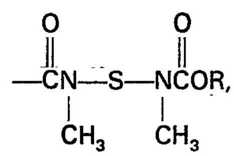, 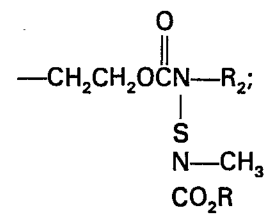;

V est O, $S(O)_n$, $CT_7T_8$;
$R_{10}$ est H, un groupe alcoyle en $C_1$—$C_3$, Cl;
$T_7$ est H, un groupe alcoyle en $C_1$—$C_2$, carboalcoxy alcoyle de 3 à 8 atomes de carbone, carboalcoxy en $C_2$—$C_4$;
$T_8$ est H ou un groupe alcoyle en $C_1$—$C_4$;
$T_7$ et $T_8$ peuvent être pris ensemble pour former un noyau carbocyclique de 5—6 atomes de carbone;
Q est un nombre de 0 à 2;
k, l, m sont chacun indépendamment un nombre de 0 à 3;
n et p sont chacun indépendamment un nombre de 0 à 2; avec les conditions suivantes:
a) la somme de k, l et m est au moins deux;
b) le nombre total d'atomes de carbone de $-(CT_1T_2)_k\,(CT_3T_4)_l\,(CT_5T_6)_m-$ ne dépasse pas 9; exceed g;

c) quand A est 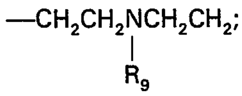 alors X et Y sont tous deux de l'oxygène et $R{=}R_1$;

d) quand A est $CT_1T_2CT_3T_4-[OCT_1T_2CT_3T_4]_p\ OCT_1T_2CT_3T_4$, X et Y sont tous deux de l'oxygène.

2. Un composé selon la revendication 1, dans lequel $R{=}R_1$.

3. Un composé selon la revendication 2, dans lequel R et $R_1$ sont tous deux

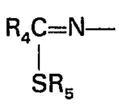 ou 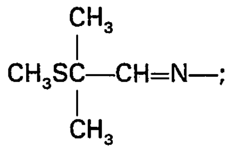; X et Y sont tous deux de l'oxygène;

A est $(CT_1T_2)_k(CT_3T_4)_l(CT_3{-}T_6)_m$ ou 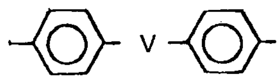; et

k, l et m sont chacun indépendamment un nombre de 0 à 3.

4. Un composé selon la revendication 3, dans lequel R et $R_1$ sont tous deux

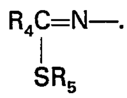.

5. Un composé selon la revendication 4, dans lequel

$R_2$, $R_3$ et $R_5$ sont des groupes méthyle;

$R_4$ est un groupe alcoyle en $C_1{-}C_2$;

A est $(CT_1T_2)_k(CT_3T_4)_l(CT_3{-}T_6)_m$ ou 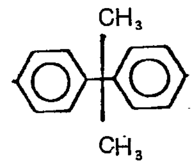; et

$T_1$ à $T_6$ sont chacun de l'hydrogène; avec la condition que la somme de k, l et m n'est pas supérieure à 5.

6. Un composé selon la revendication 1, qui est le N,N'-[[1,2-éthanediylbis[oxycarbonyl(N-méthylimino)thio(N-méthylimino)carbonyloxy]]]-bis-(éthanimidothioate) de diméthyle.

7. Un composé selon la revendication 1, qui est le N,N'-[[1,3-propanediylbis[oxycarbonyl(N-méthylimino)thio(N-méthylimino)carbonyloxy]]]-bis-(éthanimidothioate) de diméthyle.

8. Un composé selon la revendication 1, qui est le N,N'-[[2,2-propanediylbis[4,1-phénylèneoxy-carbonyl-(N-méthylimino)thio(N-méthylimino)carbonyloxy]]]bis-(éthanimidothioate) de diméthyle.

9. Un composition pour l'agriculture constituée essentiellement d'un diluant, d'un agent tensioactif ou de leurs mélanges et d'un pesticide, caractérisée en ce que le pesticide est un composé selon l'une quelconque des revendications 1 à 8.

10. Un procédé de lutte contre des insectes ou des nématodes par application à un lieu à protéger d'une quantité efficace du point de vue insecticide ou nématocide d'un pesticide, caractérisé en ce que le pesticide est un composé selon l'une quelconque des revendications 1 à 8.

11. Un procédé pour la préparation d'un composé tel que défini dans la revendication 1, selon lequel

(a) quand $R{=}R_1$, on met en contact en présence d'un accepteur d'acide

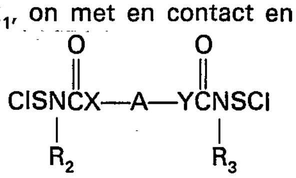 et 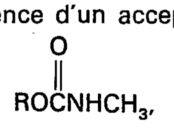, ou

(b) on met en contact en présence d'un accepteur d'acide

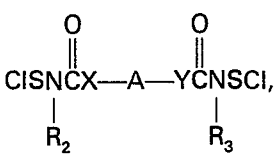, 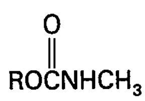 et 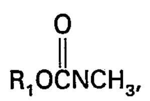, ou

(c) quand $R=R_1$ et $R_2=R_3=CH_3$, on met en contact en présence d'un accepteur d'acide

(1) HX—A—YH et 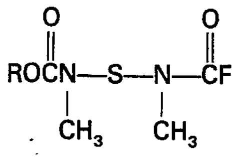 ou

(2) 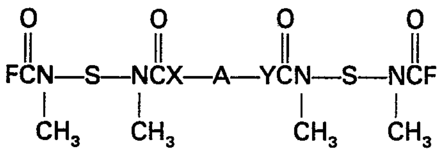 et ROH, ou

(d) quand R est différent de $R_1$ et $R_2=R_3=CH_3$, on met en contact en présence d'un accepteur d'acide

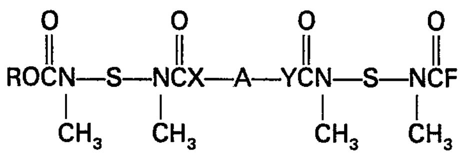 et $R_1OH$.